(19) Europäisches Patentamt
European Patent Office
Office européen des brevets

(11) Publication number: **0 200 218 B1**

(12) **EUROPEAN PATENT SPECIFICATION**

(45) Date of publication of patent specification: **22.01.92**

(51) Int. Cl.⁵: **C07D 487/22**, A61K 31/40, A61K 31/49, //(C07D487/22, 257:00,209:00,209:00,209:00, 209:00)

(21) Application number: **86105953.3**

(22) Date of filing: **30.04.86**

The file contains technical information submitted after the application was filed and not included in this specification

(54) **Tetrapyrrole therapeutic agents.**

(30) Priority: **30.04.85 US 728785**

(43) Date of publication of application: **05.11.86 Bulletin 86/45**

(45) Publication of the grant of the patent: **22.01.92 Bulletin 92/04**

(84) Designated Contracting States: **AT BE CH DE FR GB IT LI LU NL SE**

(56) References cited:
**EP-A- 0 168 831**
**EP-A- 0 168 832**
**WO-A-84/01382**

**CHEMISCHE BERICHTE, vol. 90, no. 4, April 1957, pages 470-481, Weinheim, DE; W. LAUTSCH et al.: "Über durch Porphyrincarbonsäuren n-Acylierte Aminosäuren und Über einige Funktionelle Derivate von Porphyrinen und Phorbinen der Blut- und Blatt-Farbstoffreihe"**

**TETRAHEDRON LETTERS, no. 23, 1978, pages 2017-2020, Pergamon Press Ltd, GB: A. PELTER et al.: "The structures of amino-acid conjugates of bonellin derived from the marine echuroid bonellia viridis"**

(73) Proprietor: **Nippon Petrochemicals Co., Ltd.**
**Saiwai Building, 1-3-1 Uchisaiwai-cho**
**Chiyoda-ku**
**Tokyo(JP)**

(72) Inventor: **Bommer, Jerry C.**
**Rural Route No. 2 Box 516**
**Ogden Utah(US)**
Inventor: **Burnham, Bruce F.**
**127 West 900 North**
**Logan Utah(US)**

(74) Representative: **Eitle, Werner, Dipl.-Ing. et al**
**Hoffmann, Eitle & Partner Patentanwälte Arabellastrasse 4**
**W-8000 München 81(DE)**

EP 0 200 218 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention relates to new compounds which are useful in photodiagnosis and phototherapy, especially in the detection and treatment of tumors and cancerous tissues in the human or animal body.

### Description of the Prior Art

It is known to irradiate tumors and cancerous tissues in the human body with intensive light following administration of a hematoporphyrin derivative in the wavelength range of 626 to 636 nanometers to reduce and, at times, destroy the cancerous cells (see PCT published specification WO 83/00811). It is also known that porphyrins, especially the sodium salt of protoporphyrins, can maintain or promote the normal functions of cells and are useful for preventing the genesis, growth, metastasis , and relapse of malignant tumors. Japanese Published Patent Application No. 125737/76 describes the use of porphyrins as tumor inhibiting agents, exemplifying etioporphyrin, mesoporphyrin, protoporphyrin, deuteroporphyrin, hematoporphyrin, coprophyrin, and uroporphyrin.

In Tetrahedron Letters No. 23, pp. 2017-2020 (1978), there is described an amino monocarboxylic acid adduct of the pigment bonellin obtained by extraction of principally the body wall of the marine echuroid B. viridis. The structure of these adducts is presumed to be an amide formed through either of the free carboxy groups of bonellin and the amino monocarboxylic acid. Hydrolysis of the adduct yielded a mixture of valine, isoleucine, leucine and alloisoleucine. No use for these amino acid adducts is described in this reference.

Mesoporphyrin and mesohaemin bis amino acid esters and the corresponding acids are described in Chemische Berichte, vol. 90, no. 4, 1957(pp. 470-481). Specific bis amino acid compounds include DL-valine, DL-leucine, DL-phenylalanine, DL-isoleucene and L-glutamic acid esters (and the corresponding free acids) bis-adducts of mesoporphyrin and mesohaemin. No therapeutic use of these compounds is disclosed, however.

PCT Patent Application No. WO 84/01382 describes the use of a new derivative of hematoporphyrin as useful in the localization and treatment of tumors.

EP-A-0168831 and EP-A-0168832 both published on 22nd January 1986 disclose fluorescent mono, di or polyamides of an aminodicarboxylic acid and a tetrapyrrole containing carboxy groups. The compounds are noted to be useful for the detection and/or treatment of mammalian tumors.

That the tetrapyrroles cause intense photosensitivity in animals is well-known and has been documented in numerous articles in literature, e.g., J. Intr. Sci. Vitaminol, 27, 521-527 (1981); Agric. Biol. Chem., 46(9), 2183-2193 (1982); Chem. Abst. 98, 276 (1983) and 88, 69764m (1928).

The new products contemplated by this invention are a fluorescent mono-,di- or polyamide of an aminomonocarboxylic acid and a tetrapyrrole compound of the formula:

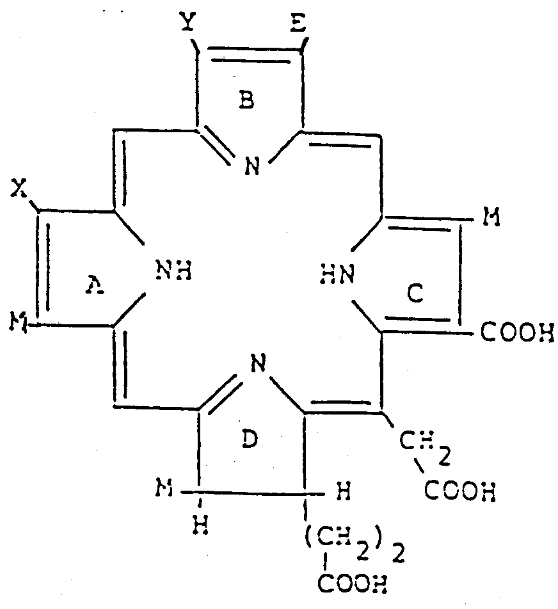

wherein

X = H, vinyl, ethyl or formyl;
Y = methyl or formyl;
M = methyl; and
E = ethyl

and pharmaceutically-acceptable salts thereof.

The cyclic tetrapyrroles of the present invention are based upon the common parent tetrapyrrole, uroporphyrinogen, and which has the following ring structure:

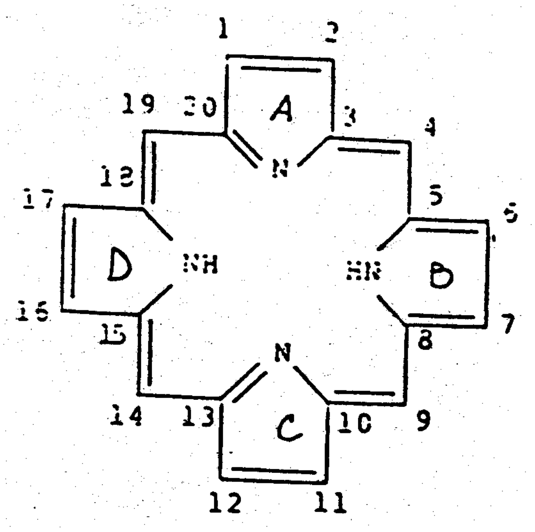

in which the positions in the molecule are numbered 1-20, and the rings identified by letters A, B, C and D. There are present in the ring system four pyrrole rings joined through the alpha positions of the respective pyrrole rings by a methine group, i.e., -CH=.

The tetrapyrroles employed in the present invention are all known or derived by various means and various alteration procedures from natural tetrapyrroles. The naturally occurring tetrapyrroles have as their common ancestor uroporphyrinogen III, a hexahydroporphyrin reduced at the bridge positions.

A further characteristic of the present new compounds is the presence of at least one amide linkage in a substituent at any of the numbered positions of the ring structure. These are present in the instant new compounds together with other substituents as defined hereinafter.

Thus, the present invention contemplates amino acid or peptide derivatives of compounds which

contain the chromophore of certain chlorins and bacteriochlorins, as well as related porphyrin compounds. The peptide linkage involves a carboxy group of the chromophore-bearing compound and the amino group of an aminomonocarboxylic acid. The present new compounds embrace derivatives of the tetrapyrroles which contain three carboxy groups. These derivatives include porphyrins of the major classes of tetrapyrroles: chlorins and bacteriochlorins, which are well-known to those skilled in this art.

The amino acid employed in the present invention to form the aforesaid peptide linkage are aminomonocarboxylic acids in which the amino group, of course, is located on a carbon atom of the carboxylic acid. The specific position of the amino group in the carbon atom chain is not critical, the only requirement being that the amino group be available to form the requisite peptide linkage with the carboxyl group of the selected porphyrin. Thus, a variety of amino monocarboxylic acids are useful in the present invention, including serine, glycine, methionine, $\alpha$ -alanine, $\beta$ -alanine, $\alpha$ -phenylalanine, $\epsilon$ -aminocaproic acid, piperidine-2-carboxylic acid, pyrrole-2-carboxylic acid, piperidine-2-propionic acid, pyrrole-2-acetic acid, lysine, threonine, cysteine, and other natural amino acids. These amino acids may be substituted with angular alkyl groups such as methyl and ethyl groups, as well as other groups which do not adversely affect the capability of the amino group to form the peptide linkage, e.g., hydroxy, alkoxy groups or acyloxy groups, and may also include additional amino groups. Preferably, the amino acid is an alpha amino acid, and further that such an alpha amino acid is polar. It is further preferred that the amide is a mono-, or diamide.

The preferred amino acids are polar amino acids, particularly the naturally occurring polar $\alpha$ -amino acids, serine, methionine, threonine and cysteine, which are readily available and, up to the present, have provided the best results. For the purpose of this invention, "polar amino acids" include the amino acids containing, in addition to requisite amino and carboxylic acid groups, an oxygen, nitrogen or sulfur-containing groups, especially oxygen-containing groups such as hydroxy, acetoxy and methoxy groups.

Exemplary compounds of the tetrapyrrole classes are illustrated in Table I in which the numbered positions of the tetrapyrrole ring structure are used to designate the position of the indicated substituent. The absence of double bonds in the ring system is designated under "dihydro" with each set of numbers (ring position) indicating the absence of a double bond between the designated positions.

TABLE I -

| PORPHYRIN | Ring Position A 1 | A 2 | B 6 | B 7 | C 11 | C 12 | C 14 | D 16 | D 17 | Dihydro |
|---|---|---|---|---|---|---|---|---|---|---|
| Chlorin $c_6$ | Me | V | Me | Et | Me | $CO_2H$ | Ac | H<br>Pr | H<br>Me | 16,17 |
| Mesochlorin $c_6$ | Me | Et | Me | Et | Me | $CO_2H$ | Ac | H<br>Pr | H<br>Me | 16,17 |
| 2-Desvinylchlorin $c_6$ (or Deuterochlorin $c_6$) | Me | H | Me | Et | Me | $CO_2H$ | Ac | H<br>Pr | H<br>Me | 16,17 |
| 2-Formylchlorin $c_6$ | Me | CHO | Me | Et | Me | $CO_2H$ | Ac | Pr<br>H<br>Pr | Me<br>H<br>Me | 16,17 |
| Rhodin $g_7$ | Me | V | CHO | Et | Me | $CO_2H$ | Ac | H<br>Pr | H<br>Me | 16,17 |

Notes:

| | | |
|---|---|---|
| Me: | $-CH_3$ | (Methyl group) |
| Pr: | $-CH_2CH_2COOH$ | (Propionic acid group) |
| V: | $-CH=CH_2$ | (Vinyl group) |
| Et: | $-CH_2CH_3$ | (Ethyl group) |
| Ac: | $-CH_2COOH$ | (Acetic acid group) |
| ACL: | $CH_3-CO-$ | (Acetyl group) |

Particularly preferred compounds of this invention include:

Chlorin Derivatives

Mono, di and triserinyl chlorin $e_6$

Mono, di and triserinyl mesochlorin $e_6$
Mono, di and trithreoninyl chlorin $e_6$
Mono, di and trithreoninyl chlorin $e_6$
Mono, di and triglycyl acetylchlorin $e_6$
Mono, di and triserinyl rhodin $g_7$
Mono, di and trimethionyl formylchlorin $e_6$
Mono, di and trithreoninyl rhodin $g_7$
Mono, di and tricysteinyl chlorin $e_6$
Mono, di and tricysteinyl rhodin $g_7$

Bacteriochlorin Derivatives

Mono, di and triserinyl bacteriochlorin $e_6$
Mono, di and trithreoninyl bacteriochlorin $e_6$
Mono, di and tricysteinyl bacteriochlorin $e_6$
Further preferred compounds include:
mono- or di-L-serinyl chlorin $e_6$,
mono- or di-L-serinyl bacteriochlorin $e_6$,
mono- or di-L-cysteinyl bacteriochlorin $e_6$,
mono- or di-L-serinyl rhodin $g_7$,
mono- or di-L-cysteinyl rhodin $g_7$,
mono- or di-L-cysteinyl mesochlorin $e_6$,
mono- or di-L-serinyl mesochlorin $e_6$,
mono- or dialanyl chlorine $e_6$,
dileucyl chlorin $e_6$,
di-methionyl chlorin $e_6$,
mono- or diglycyl chlorin $e_6$,
mono- or dithreoninyl chlorin $e_6$
mono- or dicysteinyl chlorin $e_6$,
dityrosyl chlorin $e_6$,
diarginyl chlorin $e_6$,
dihistidyl chlorin $e_6$, or
mono- or di-$\epsilon$-amino-caproyl chlorin $e_6$, or pharmaceutically-acceptable salts thereof.

The present new compounds form salts with either acids or bases. The acid salts are particularly useful for purification and/or separation of the final amide products as are the salts formed with bases. The base salts, however, are particularly preferred for diagnostic and therapeutic use as hereindescribed.

The acid salts, are formed with a variety of acids such as the mineral acids, hydrochloric, hydrobromic, nitric and sulfuric acids, organic acids such as toluenesulfonic and benezenesulfonic acids.

The base salts include, for example, sodium, potassium, calcium, magnesium, ammonium, triethylammonium, trimethylammonium, morpholine and piperidine salts and similar such salts.

The acid and base salts are formed by the simple expediency of dissolving the selected amino acid tetrapyrrole amide in an aqueous solution of the acid or base and evaporation of the solution to dryness. The use of a water-miscible solvent for the amide can assist in dissolving the amide.

The final amide products can also be converted to metal complexes for example by reaction with metal salts. The magnesium complexes may be useful for the same purpose as the adduct product. Other metal complexes, as well as the magnesium complex, including, for example, iron and zinc, are useful to preclude contamination during processing of the adduct product by metals such as nickel, cobalt and copper, which are difficult to remove. Zinc and magnesium are readily removed from the final adduct product after processing is completed.

Since many of the aminodicarboxylic acids exist in both the D- and L-forms, and also are employed in mixtures of these forms as well as the D,L-form, the selection of the starting amino acid well, of course, result in products in which the respective isomer or mixture of isomers exist. The present invention contemplates the use of all such isomers, but the L-form is particularly preferred.

The present new compounds are prepared by the usual peptide synthetic routes which generally include any amide-forming reaction between the selected amino acid and the specific tetrapyrrole. Thus, any amide-forming derivative of the tetrapyrrole carboxylic acid can be employed in producing the present new peptides, e.g., lower alkyl esters, anhydrides and mixed anhydrides.

The preferred preparative methods use mixed anhydrides of the carboxylic acid or carbodiimides. The

reactants are merely contacted in a suitable solvent therefor and allowed to react. Temperatures up to the reflux temperature can be used, with the higher temperatures merely reducing the reaction time. Excessively high temperatures are usually not preferred so as to avoid unwanted secondary reactions however.

The procedures for forming the instant peptides are well known in this art and are provided in detail in the accompanying examples.

When the selected tetrapyrrole contains at least three carboxyl groups, then mixtures of products can be formed including isomeric monopeptide products and di- and even tri- or higher peptide products, depending on the number of carboxyl groups and depending on the selected stoichiometry. Thus, when equimolar mixtures of amino acid and tetrapyrrole are reactd, not only monopeptides but also dipeptides are obtained, although the monopeptide would predominate. With higher molar ratios, the nature of the products will similarly vary. It is generally possible to separate the monopeptides and higher peptides using known chromatographic techniques. However, such separations are not necessary since the mixed peptides are usually comparable to the separated products in their ultimate use. Thus, mixtures of the mono-, di- and tri-peptides of the same tetrapyrrole can be used.

Usually, unreacted tetrapyrrole is separated from the peptide products of the invention during purification as, for example, by chromatographic techniques.

## Photodiagnosis and Phototherapy

The compounds of the present invention are useful for the photodiagnosis and phototherapy of tumor, cancer and malignant tissue (hereinafter referred to as "tumor").

When a man or animal having tumor is treated with doses of a compound of the present invention and when appropriate light rays or electromagnetic waves are applied, the compound emits light, i.e., fluorescence. Thereby the existence, position and size of tumor can be detected, i.e., photodiagnosis.

When the tumor is irradiated with light of proper wavelength and intensity, the compound is activated to exert a cell killing effect against the tumor. This is called "phototherapy".

Compounds intended for photodiagnosis and phototherapy ideally should have the following properties:

(a) non-toxic at normal therapeutic dosage unless and until activated by light;
(b) should be selectively photoactive;
(c) when light rays or electromagnetic waves are applied, they should emit characteristic and detectable fluorescence;
(d) when irradiated with light rays or electromagnetic waves are applied, they are activated to an extent to exert a cell killing effect against tumor; and
(e) easily metabolized or excreted after treatment.

In accordance with testing up to the present, the present new compounds have the foregoing properties and are also characterized by reasonable solubility in saline at physiological pH.

The present new compounds possess greater fluorescence in tumors than do the corresponding basic tetrapyrroles. Their use provides the best contrast in tumors compared to normal tissue around the tumor. The instant compounds absorb activating energy for phototherapy in the convenient range of 600 to 800 nanometers, with the preferred compounds absorbing in the 620-760 nanometer range, i.e., light of longer wavelengths which more readily permits penetration of energy into the tumor for phototherapeutic purpose.

In present experience, the present compounds more uniformly distribute throughout the tumor than the basic tetrapyrrole permitting the use of considerably lower dosage (to about 1/10th of the required normal dose of the basic tetrapyrrole) which lessens, if not eliminates, photosensitization in the host. They also possess a more consistent fluorescence whereas some of the corresponding tetrapyrroles show inconsistent fluorescence or the fluorescence varies from day to day in the host.

A particularly advantageous property of the present compounds resides in the ease with which they are excreted by the host. Generally, within 24 to 72 hours of intravenous or intraperitonal administration, there are little or no detectable amounts in normal muscle tissue. Up to about 50% of the present compounds are recovered from the feces of the host within 24-72 hours of injection whereas under equivalent circumstances, substantial amounts of the corresponding tetrapyrroles remain, and up to about 20% of peptides formed with amino monocarboxylic acids remain. This property is extremely important in that it contributes to minimization of photosensitization of the host.

The instant compounds can be used for diagnosis and therapeutic treatment of a broad range of tumors. Examples of tumors are gastric cancer, enteric cancer, lung cancer, breast cancer, uterine cancer, esophageal cancer, ovarian cancer, pancreatic cancer, pharyngeal cancer, sarcomas, hepatic cancer, cancer of the urinary bladder, cancer of the upper jaw, cancer of the bile duct, cancer of the tongue, cerebral tumor, skin cancer, malignant goiter, prostatic cancer, cancer of the parotid gland, Hodgkins's disease,

multiple myeloma, renal cancer, leukemia, and malignant lymphocytoma. For diagnosis, the sole requirement is that the tumor be capable of selectively fluorescing when exposed to proper light. For treatment, the tumor must be penetrable by the activation energy. For diagnosis, light of shorter wavelength is used whereas for therapeutic purposes light of longer wavelength is used to permit ready penetration of the tumor tissue. Thus, for diagnosis, light of from 360-760 nanometers can be used, and for treatment, from 620 to 760, depending on the individual characteristics of the tetrapyrrole. The absorption characteristics of the present new compounds are substantially the same as the tetrapyrrole from which derived.

It is necessary that the light rays be so intense as to cause the compounds to emit fluorescence for diagnosis and to exert a cell killing effect for therapy.

The source of irradiation for photodiagnosis and phototherapy is not restricted, however, but the laser beam is preferable because intensive light rays in a desired wavelength range can be selectively applied. For example, in photodiagnosis, the compound of the invention is administered to a human or animal body, and after a certain period of time, light rays are applied to the part to be examined. When an endoscope can be used for the affected part, such as lungs, gullet, stomach, womb, urinary bladder or rectum, it is irradiated using the endoscope, and the tumor portion selectively emits fluorescence. This portion is observed visually, or observed through an adapted fiber scope by eye or on a CRT screen.

In phototherapy, after administration of the dosage, the irradiation is carried out by laser beams from the tip of quartz fibers. Besides the irradiation of the surface of tumor, the internal part of the tumor can be irradiated by inserting the tip of quartz fibers into the tumor. The irradiation can be visually observed or imaged on a CRT screen.

For photodiagnosis, light of wavelengths between 360 and 760 nm. is suitable for activating the present tetrapyrrole compounds. Of course, each compound has a specific optimal wavelength of activation. A long wavelength ultraviolet lamp is particularly suitable for photodiagnosis. Similar methods for viewing of the treated tumor can he used as already described for phototherapy.

The dosages of the present new compounds will vary depending on the desired effect, whether for diagnosis or for treatment. For diagnosis, doses of as little as 1 mg/kg will be effective, and up to about 7.5 mg/kg can be used. For treatment, the dose will usually approximate about 0.5 mg/kg. Of course, the dosage for either diagnosis or treatment can be varied widely in view of aforesaid advantageous properties of the present compounds, e.g., the ease of elimination from the host, for one.

The present compounds are apparently non-toxic at the dosage levels employed for diagnosis or treatment. No mortality of test animals due the present compounds has been noted in studies employing dosage levels up to 20 mg/kg.

For both diagnosis and treatment, the present compounds can be administered by the oral, intravenous, or intramuscular routes. They can be formulated as lyophilized sterile, pyrogen-free compounds, preferably in the form of basic salts, e.g., sodium salt. The preferred dosage forms are provided as injectable solutions (isotonic).

The irradiation source used in treatment of tumors containing compounds of this invention is a filtered, high-intensity, continuous source or pumped dye, or other laser and light delivery system, which is capable of performing within the following limits: power intensity 20-500 mw/cm$^2$ at wavelengths between 620 and 680 nm. and a total output of at least 4 watts or greater. Several currently commercially available lasers meet these criteria.

The tetrapyrroles can be prepared by various synthetic methods which are found in the literature, e.g.,

<u>Chlorin $e_6$</u>

Willstatter, R., Stoll, A.; <u>Investigations on Chlorophyll,</u> (Trans., Schertz, F.M., Merz, A.R.,) p. 176. Science Printing Press, Lancaster, Pennsylvani, 1928.
Willstatter, R., Isler, M.; <u>Ann. Chem.</u>, 390, 269 (1912).
Fisher, H., Baumler, R.; <u>Ann. Chem.</u>, 474, 65 (1929).
Fisher, H., Siebel, H.; <u>Ann. Chem.</u>, 499, 84 (1932).
Conant, J.B., Mayer, W.W.; <u>J. Amber. Chem. Soc.</u>, 52, 3013 (1930).

<u>Chlorin $e_6$, $e_4$, mesochlorin $e_6$, bacteriochlorin $e_6$</u>

Fischer and Orth, "Des Chemie des Pyrrole" Akademische Verlazsgesellschaft, Leipzig, 1940, Vol. II, Part 2.

<u>General Reference for Porphyrins</u>

"Porphyrins and Metalloporphyrins" ed. Kevin M. Smith, Elsevier 1975 N.Y.

The compounds of the present invention can be administered to the host in a variety of forms adapted to the chosen route of administration, i.e., orally, intraveneously, intramuscularly or subcutaneous routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabons as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporanous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The present new compounds may also be applied directly to tumors, whether internal or external, in the host in topical compositions. Exemplary compositions include solutions of the new compounds in solvents, particularly aqueous solvents, most preferably water. Alternatively, for topical application particularly to skin tumors, the present new compounds may be dispersed in the usual cream or salve formulations commonly used for this purpose or may be provided in the form of spray solutions or suspensions which may include

a propellant usually employed in aerosol preparations.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatable with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of tumors in living subjects.

The following examples further illustrate the invention.

EXAMPLE 1

L-Monoserinyl chlorin $e_6$ (carbodiimide method)

METHOD I

150 mg of chlorin $e_6$ and 250 mg of L-serine t-butyl ester hydrochloride are dissolved in 20 ml of dimethyl formamide. There is made a total of 3-100 mg additions of N,N'-dicyclohexyl-carbodiimide at one hour intervals. After 4 hours, the reaction mixture is diluted with 300 ml ether, washed twice with 200 ml $H_2O$ then extracted with 40 ml 1 M KOH. The KOH solution is allowed to hydrolyze overnight and then heated to 70 ° C. for 10 minutes.

The pH of the solution is adjusted to 7, and then any residual ether is removed by flash evaporation. The solution is then applied to a reverse phase (C-18 silica) column (1.5 cm x 30 cm), and purified by a stepwise elution of methanol/.01 m pH 6.85 $KPO_4$ buffer, eluted with 5% methanol until unwanted polar pigments were removed. Monoserinyl chlorin $e_6$ is eluted off with 6-8% methanol, and unreacted chlorin $e_6$ removed with 25% methanol.

The product is precipitated at pH 3 after flash evaporating briefly to remove methanol, and then washed at the centrifuge 3 times with dilute acetic acid.

The product is dried under vacuum.

METHOD II

Chlorin $e_6$ was prepared according to the procedure of Fischer and Stern, Di Chemie Des Pyrroles, Volume II, second half, Leipsig 1940, Akademische Verlagsgesellschaft, pp. 91-93.

100 mg of the chlorin $e_6$ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room temperature for 2 hours. At this time 0.5 ml of glacial acetic acid was added, then 30 ml of methanol and 12 ml of $H_2O$.

The solution was applied to a C-18 reverse phase column (14 x 2 cm). The column was washed with $H_2O$ (100 ml) then 4 ml of 1M $NH_4OH$, then with $H_2O$ again (50 ml). Eluted product with MeOH/$H_2O$. Fractions eluted from the column with 30% to 80% MeOH contained product as well as carbodiimide activated chlorin as determined by TLC on C-18 reverse phase plates with solvent 70% MeOH/30% buffer (0.1M sodium phosphate pH 6.85) V/V.

These fractions were pooled and enough 3 N NaOH was added to make the solution 0.1N in NaOH. After 1 hour, the hydrolysis was complete as determined by TLC in the above system. Removed the methanol by rotary evaporation and adjusted the pH of the solution to 7.5 with HCl. The chlorin solution was then reapplied to the same reverse phase column, washed with water, and eluted with MeOH/water using a stepwise gradient from 10 to 50% methanol. The fractions containing pure mono-L-serinyl chlorin as determined by TLC ($R_f$ slightly greater than the unsubstituted chlorin) were pooled, the methanol removed by rotary evaporation, and the product dried as the trisodium salt by lyophylization.

EXAMPLE 2

Mono and Di (L) Serinyl Chlorin $e_6$ (Carbodiimide Method)

400 mg of chlorin $e_6$ and 1 g of L-serine benzyl ester p-tosylate are dissolved in 75 ml of dimethylformamide. Temperature of the solution is maintained at 65-70° C. with stirring and 100 mg of N,N'-dicyclohexyl carbodiimide is added. (A total of 3 additions are made at 2 hour intervals). The solution is allowed to stir at this temperature for a total of 20 hrs., then checked by TLC (reverse phase) (C-18 silica) plate, 70% methanol, 30% .01 M pH 6.85 $KPO_4$ buffer. The TLC shows greater than 50% mono-substitution with some di-substitution.

150 ml of ether is added, and agitated with 100 ml of water and several drops of glacial acetic acid. The ether phase is separated and the aqueous phase extracted several more times with 100 ml of ether. The ether extracts are combined and washed with water (100 ml) four times to remove dimethylformamide.

The serinyl chlorin $e_6$ esters are then extracted into 100 ml of 1 M KOH (4 extractions of 25 ml each). The KOH solution is allowed to stand at ambient temperat7re for 24 hours to hydrolyze. The components are separated by neutralizing the solution of pH 7 and applying to a reverse phase (C-18 silica) column (1.5 cm x 30 cm). The elution is performed using a 1 liter gradient of 30% mthanol to 80% methanol with 0.1 M pH 6.85 $KPO_4$ buffer. Fractions are collected and characterized by TLC. The order of elution is di(L) diserinyl chlorin $e_6$, L-monoserinyl chlorin $e_6$ and chlorin,$e_6$. Methanol is removed is flash evaporated and the individual components precipitated at pH 3, using HCl.

The products are collected by centrifugation, washed several times with very dilute acetic acid and dried under vacuum.

EXAMPLE 3

Mono Glycyl Chlorine $e_6$ (Mixed Anhydride Method)

625 mg of chlorin $e_6$ was dissolved in 300 ml of dimethyl formamide (DMF) and 277 µl (0.002 moles) of triethylamine (TEA) was added to the DMF solution. After stirring for five minutes, 201 µl (0.002 moles) of ethylchloroformate (EC) was added and stirred for 1 1/2 hours at room temperature.

75 mg (.0009 moles) of glycine (ammonia free) was added to the DMF solution and allowed to stir three hours at 50-60° C.

The DMF solution was tested for product by reverse phase (C-18 silica) TLC using methanol/0.01M sodium phosphate buffer, pH 6.85, 70/30, to develop the chromatogram.

The DMF solution was flashed to near dryness, then dissolved in dilute NaOH and the pH adjusted to 2.5-3 to precipitate the solid. The precipitate was then placed on a reverse phase (C-18 silica) column 3.7 cm x 45 cm.

Fractions were eluted, using 20-40% methanol in 0.01 M sodium phosphate buffer, pH 6.85. The fractions were pooled according to individual components.

The methanol was flashed off and the material was precipitated at pH 2.5-3.0. The precipitate was washed and centrifuged 3 times in dilute acetic acid in water. The product was dried under vacuum. The yield of mono glycyl chlorin $e_6$ was 87.5 mg.

EXAMPLE 4

Preparation of Mono-L-Asparaginyl Chlorin $e_6$

500 mg of chlorin $e_6$ and 175 mg of 1-ethyl-3-(3-dimethylamine-propyl) carbodiimide hydrochloride were dissolved in 10 ml of N, N'-dimethyl formamide. After 5 minutes, 410 mg of L-asparagine was added. The solution was agitated for 4 hours, The asparagine did not dissolve totally during this reaction, but reverse phase (C-18) TLC 70/30 MeOH/.01M sodium phosphate buffer pH 6.85 showed some product at this time, ($R_f$ slightly greater than chlorin $e_6$). The reaction was terminated by adding 2.5 ml glacial acetic acid, then diluting to a total volume of 100 ml with Methanol, then adding 25 ml of $H_2O$ slowly, with stirring. The solution was then applied to a 14 x 2 cm reverse phase (C-18) column, washed with water then with 5 ml of 0.1M NaOH, finally with 50 ml of 0.01 M sodium phosphate buffer, pH 6.85. The product was eluted off with MeOH/$H_2O$ in a stepwise gradient from 20% MeOH to 50% MeOH. The fractions containing pure mono-L-asparaginyl chlorin $e_6$, as determined by TLC using the conditions stated above, were pooled, and the methanol removed by rotary evaporation. The product was isolated as the trisodium salt by lyoph-

lization.

EXAMPLE 5

L-Monocysteinyl chlorin $e_6$ (carbodiimide method)

METHOD I

130 mg of chlorin $e_6$ and 260 mg L-cysteine methyl ester hydrochloride are dissolved in 18 ml of dimethyl formamide. 100 mg of N,N'-dicyclohexylcarbodiimide is added and the reaction mixture stirred for 1 hour. 50 mg more carbodiimide is then added. After 1 hour, the reaction mixture appears to contain 75-80% of the monosubstituted product by reverse phase TLC (C-18 plates with 70% MeOH, 30% .01 M $KPO_4$ pH 6.85). 200 ml Diethyl ether was added, washed twice with 100 ml $H_2O$, then extracted with 30 ml 1 M KOH.

The product is allowed to hydrolyze in the dark in the KOH solution for 12 hours, then is heated to 70°C for 10 minutes, to complete the hydrolysis of the ester groups. The product is then separated by reverse phase column chromatography (C-18 reverse phase silica 1.5 cm x 30 cm), using stepwise gradient elution with methanol in buffer .01 M $KPO_4$ pH 6.85. 5% Methanol removed polar impurities. Chlorin $e_6$ is eluted off the column with 25% methanol. The methanol is removed by flash evaporation and the L-monocysteinyl chlorin $e_6$ is precipitated at pH 3, collected and washed 3 times at the centrifuge with dilute acetic acid, and dried under vacuum.

METHOD II

300 mg of chlorin $e_6$ and 105 mg of 1-ethyl-3-(3-dimethylamine-propyl)carbodiimide hydrochloride was dissolved in 6 ml of N, N'-dimethylformamide. After 5 minutes, 255 mg of L-cysteine hydrochloride was added. The solution was stirred at toom temperature for 5 hours. The rest of the procedure is the same as for the preparation of mono-L-asparaginyl chlorin $e_6$.

EXAMPLE 6

Preparation of Mono-L-Serinyl-2- formylchlorin $e_6$ (Mono-L-Serinyl 2-Desvinyl-2-Formyl-Chlorine $e_6$)

500 mg of chlorin $e_6$ trimethyl ester was prepared according to the procedure of Fisher and Stern, in Di Chemie Des Pyrroles, Volume II, second half, Leipsig 1940, Akademische Verlagsgesellschaft, pp. 98-102. The chlorin $e_6$ trimethyl ester was dissoved in 600 ml of refluxing acetone. 400 mg of Potassium permanganate and 800 mg of magnesium sulfate dissolved in 130 ml of $H_2O$ were added slowly over approximately a one hour period to the refluxing acetone solution. The solution was allowed to reflux for 1/2 hour after addition was complete. After cooling, 300 ml of methylene chloride was added, and the mixture was washed 3 times with water in a separatory funnel. The volume of methylene chloride was reduced and the product chromatographed on silica gel, eluting with a gradually increasing percentage of ethyl acetate in the $CH_2Cl_2$. The first major brown band which eluted was collected as the product, 2-Desvinyl-2-Formyl-Chlorin $e_6$. Yield 94 mg.

The product was saponified by dissolution in refluxing n-propanol (0.1 ml/mg) and addition of 6 fold equivalent of 1N KOH. The tripotassium salt was filtered off, washed with n-propanol and dried under vacuum, forming 2-formyl chlorin $e_6$.

100 mg of the 2-formyl chlorin $e_6$ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room stemperature for 2 hours. At this time 0.5 ml of glacial acetic acid was added, then 30 ml of methanol and 12 ml of $H_2O$.

The solution was applied to a C-18 reverse phase column (14 x 2 cm). The column was washed with $H_2O$ (100 ml) then 4 ml of 1M $NH_4OH$, then with $H_2O$ again (50 ml). Eluted product with $MeOH/H_2O$. Fractions eluted from the column with 30% to 80% MeOH contained product as well as carbodiimide activated chlorin as determined by TLC on C-18 reverse phase plates with solvent 70% MeOH/30% buffer (0.1M sodium phosphate pH 6.85) V/V.

These fractions were pooled and enough 3 N NaOH was added to make the solution 0.1N in NaOH. After 1 hour, the hydrolysis was complete as determined by TLC in the above system. Removed the

methanol by rotary evaporation and adjusted the pH of the solution to 7.5 with HCl. The chlorin solution was then reapplied to the same reverse phase column, washed with water, and eluted with MeOH/water using a stepwise gradient from 10 to 50% methanol. The fractions containing pure mono-L-serinyl chlorin as determined by TLC ($R_f$ slightly greater than the unsubstituted chlorin) were pooled, the methanol removed by rotary evaporation, and the product dried as the trisodium salt by lyophylization.

## EXAMPLE 7

### Preparation of Mono-L-Serinyl-Deuterochlorin $e_6$ (Mono-L-Serinyl 2-desvinyl-chlorin $e_6$)

A. Deuterochlorin $e_6$

Deuterchlorin $e_6$ trimethyl ester was prepared according to the procedure in Fisher and Stern in Di Chemie Des Pyrroles, Volume II, second half, Leipsig 1940, Akademische Verlagsgesellschaft, p. 104. The trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vaccum.

B. Mono-L-Serinyl Deuterochlorin $e_6$

100 mg of the deuterchlorin $e_6$ (free acid form) and 35 mg of 1-ethyl-3-(3-dimethylaminopropyl) carbodiimide hydrochloride were dissolved in 2 ml of N, N' dimethyl formamide. After 5 minutes, 125 mg of L-serine benzyl ester hydrochloride was added, stirred vigorously until solution was complete, then allowed to stand at room temperature for 2 hours. At this time 0.5 ml of glacial acetic acid was added, then 30 ml of methanol and 12 ml of $H_2O$.

The solution was applied to a C-18 reverse phase column (14 x 2 cm). The column was washed with $H_2O$ (100 ml) then 4 ml of 1M $NH_4OH$, then with $H_2O$ again (50 ml). Fluted product with MeOH/$H_2O$. Fractions eluted from the column with 30% to 80% MeOH contained product as well as carbodiimide activated chlorin as determined by TLC on C-18 reverse phase plates with solvent 70% MeOH/30% buffer (0.1M sodium phosphate pH 6.85) V/V.

These fractions were pooled and enough 3 N NaOH was added to make the solution 0.1N in NaOH. After 1 hour, the hydrolysis was complete as determined by TLC in the above system. Removed the methanol by rotary evaporation and adjusted the pH of the solution to 7.5 with HCl. The chlorin solution was then reapplied to the same reverse phase column, washed with water, and eluted with MeOH/water using a stepwise gradient from 10 to 50% methanol. The fractions containing pure mono-L-serinyl chlorin, as determined by TLC ($R_f$ slightly greater than the unsubstituted chlorin) were pooled, the methanol removed by rotary evaporation, and the product dried as the trisodium salt by lyophylization.

## EXAMPLE 8

### Preparation of Mono-L-Serinyl-2 acetyl-chlorin $e_6$ (Mono-L-Serinyl-2-desvinyl-2-acetyl chlorin $e_6$)

A. 2-acetyl chlorin $e_6$

2-acetyl chlorin $e_6$ trimethyl ester was prepared according to the procedure of Fischer and Stern, Di Chemie Des Pyrroles, Volume II, second half, Leipsig 1940, Akademische Verlagsgesellschaft, p. 185. The trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vaccum.

E. L-serinyl-2-acetyl chlorin $e_6$

100 mg of the 2-acetyl chlorin $e_6$ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room temperature for 2 hours. At this time 0.5 ml of glacial acetic acid was added, then 30 ml of methanol and 12 ml of $H_2O$.

The solution was applied to a C-18 reverse phase column (14 x 2 cm). The column was washed with

$H_2O$ (100 ml) then 4 ml of 1M $NH_4OH$, then with $H_2O$ again (50 ml). Eluted product with MeOH/$H_2O$. Fractions eluted from the column with 30% to 80% MeOH contained product as well as carbodiimide activated chlorin as determined by TLC on C-18 reverse plates with solvent 70% MeOH/30% buffer (.01M sodium phosphate, pH 6.85) V/V.

These fractions were pooled and enough 3N NaOH was added to make the solution 0.1N in NaOH. After 1 hour, the hydrolysis was complete as determined by TLC in the above system. Removed the methanol by rotary evaporation and adjusted the pH of the solution to 7.5 with HCl. The chlorin solution was then reapplied to the same reverse phase column, washed with water, and eluted with MeOH/water using a stepwise gradient from 10 to 50% methanol. The fractions containing pure mono-L-serinyl chlorin as determined by TLC ($R_f$ slightly greater than the unsubstituted chlorin) were pooled, the methanol removed by rotary evaporation, and the product dried as the trisodium salt by lyophylization.

## EXAMPLE 9

### Preparation of Mono-L-Serinyl mesochlorin $e_6$

#### A. Mesochlorin $e_6$

Mesochlorin $e_6$ trimethyl ester was prepared according to the procedure of Fischer and Stern, Di Chemie Des Pyrroles, Volume II, second half, Leipsig 1940, Akademische Verlagsgesellschaft p. 102.

The mesochlorin $e_6$ trimethyl ester was then hydrolyzed to the free acid state by dissolution in refluxing n-propanol (0.1 ml/mg) and adding 6 fold equivalent amounts of 1N KOH. The product was collected by filtration, after cooling, as the potassium salt and dried under vacuum.

#### B. Mono-L-Serinyl Mesochlorin $e_6$

100 mg of the mesochlorin $e_6$ (free acid form) and 35 mg of 1-ethyl-3-(3-dimethylaminopropyl) carbodiimide hydrochloride were dissolved in 2 ml of N, N'-dimethyl formamide. After 5 minutes, 125 mg of L-serine benzyl ester hydrochloride was added, stirred vigorously until solution was complete, then allowed to stand at room temperature for 2 hours. At this time 0.5 ml of glacial acetic acid was added, then 30 ml of methanol and 12 ml of $H_2O$.

The solution was applied to a C-18 reverse phase column (14 x 2 cm). The column was washed with $H_2O$ (100 ml) then 4 ml of 1M $NH_4OH$, then with $H_2O$ again (50 ml). Eluted product with MeOH/$H_2O$. Fractions eluted from the column with 30% to 80% MeOH contained product as well as carbodiimide activated chlorin as determined by TLC on C-18 reverse phase plates with solvent 70% MeOH/30% buffer (.01M sodium phosphate $_4$pH 6.85) V/V.

These fractions were pooled and enough 3 N NaOH was added to make the solution 0.1N in NaOH. After 1 hour, the hydrolysis was complete as determined by TLC in the above system. Removed the methanol by rotary evaporation and adjusted the ph of the solution to 7.5 with HCl. The chlorin solution was then reapplied to the same reverse phase column, washed with water, and eluted with MeOH/water using a stepwise gradient from 10 to 50% methanol. The fractions containing pure mono-L-serinyl chlorin was determined by TLC ($R_f$ slightly greater than the unsubstituted chlorin) were pooled, the methanol removed by rotary evaporation, and the product dried as the trisodium salt by lyophylization.

## EXAMPLE 10

### Di-(D,L) serinyl rhodin $g_7$ (Carbodiimide method)

140 mg of rhodin $g_7$ and 200 mg of (DL) serine methyl ester hydrochloride are dissolved in 30 ml of dimethyl formamide. 300 mg of N,N'-dicyclohexyl-carbodiimide is added. The reaction is allowed to stand for one hour, then another 300 mg of carbodiimide is added. This procedure is repeated twice and then the reaction mixture is allowed to stand overnight. The reaction may be monitored by thin layer chromatography on silica, using solvent benzene/methanol/88% formic acid 8.5/1.5/0.13 V/V/V.

The disubstituted rhodin $g_7$ has the the highest $R_f$ value, the unsubstituted rhodin $g_7$ has the lowest, with the monosubstituted isomers in between and unresolved.

After standing overnight, the reaction mixture appears to contain at least 50% of the disubstituted rhodin $g_7$. The solvent is removed under vacuum and the remaining solid dissolved in 50 ml of 3N HCl.

The solution is allowed to stand at room temperature for 48 hours to hydrolyze the ester groups, then

the chlorin mixture is precipitated at pH 2.5-3 and collected and washed with water at the centrifuge.

The rhodin g7 mixture is purified by dissolving in 0.05 M $NH_4OH$ and applying to a reverse phase (C-18 silica) column 2.5 cm x 30 cm. The solution procedure is a linear gradient from 40 to 70% methanol in 0.01 M $KPO_4$ buffer pH 6.85 (1 liter total volume).

The leading rhodin g7 is collected and flash evaporated to remove the methyl alcohol, the solution then precipitated at pH 2.5-3 and collected and washed 3 times at the centrifuge with dilute acetic acid. The product is dried under vacuum.

EXAMPLE 11

Di and Mono (L) serinyl rhodin g7 (mixed anhydride method)

50 mg (0.000087 moles) of rhodin g7 is dissolved in 100ml of tetrahydrofuran (THF) .210 1 (0.002 moles) of triethylamine is added with stirring. After 10 minutes, 195 µl (0.00179 moles) of ethylchloroformate is added. After stirring 10 minutes, 50 ml (0.01 moles) of 0.2 M KOH containing 250 mg (0.00169 moles) of (L) serine is added dropwise with stirring to the THF solution. This mixture is stirred 60 minutes at room temperature.

The organic solvent is flashed off and the reaction mixture is checked by silica TLC for product. Benzene/methanol/88% formic acid (8.5/1.5/0.13) is used to develop the chromatogram.

After checking for product, the solution is adjusted to pH 7.5-8.0 and placed on a reverse phase (C-18 silica) column 2.5 x 30 cm. The reaction mixture is resolved using a linear gradient of 40-80% methanol in 0.01 M $KPO_4$ buffer pH 6.85 (1 liter total volume).

The column effluent is collected via fraction collector and the tube contents were pooled according to individual components. The order of elution is di (L)-serinyl rhodin g7 , mono (L) serinyl rhodin g7 and unsubstituted rhodin g7.

The methanol is flashed off and the material is precipitated at pH 2.5-3.0. The precipitate is washed 3 times with dilute acetic acid in water and the product dried under vacuum.

Utilizing the aforementioned carbodiimide or the mixed anhdyride methods, the following compounds of this invention can be synthesized: (D,L)-Serinyl mesochlorin e6

Glycyl chlorin e6
Glycyl mesochlorin e6
α -(D,L)-Alanyl chlorin e6
α -(D,L)-Alanyl mesochlorin e6
β -Alanyl chlorin e6
β -Alanyl mesochlorin e6
ε -Amino-n-caproyl chlorin e6
ε -Amino-n-caproyl mesochlorin e6
(D,L)-Serinyl bacteriochlorin e6
Glycyl bacteriochlorin e6
α -(D,L)-Alanyl bacteriochlorin e6
β -Alanyl bacteriochlorin e6
ε -Amino-n-caproyl bacteriochlorin e6
Mono, di and triserinyl chlorin e6
Mono, di and triserinyl mesochlorin e6
Mono, di and trithreoninyl chlorin e6
Mono, di and trithreoninyl mesochlorin e6
Mono and diglycyl acetylchlorin e6
Mono and diserinyl rhodin g7
Mono and dimethionyl formylchlorin e6
Mono and dithreoninyl rhodin g7
Mono and dicysteinyl chlorin e6
Mono, di and triserinyl bacteriochlorin e6
Mono, di and trithreoninyl bacteriochlorin e6
Mono, di and tricysteinyl bacteriochlorin e6

EXAMPLE 12

650 mg chlorin e6 was dissolved in 30 ml of dimethylformamide (DMF). 277 µl (0.002 moles) of

triethylamine was added to the DMF solution. After stirring for five minutes, 201 ul (0.002 moles) of ethyl chloroformate was added and stirring was continued for an additional 30 minutes. 0.95 g (0.009 moles) of L-$\alpha$ -serine was added to the DMF solution and allowed to stir for one hour at 50-60 ° C.

The DMF soltuion was checked for product formation by reverse phase (C-18 silica) TLC using methanol/0.01 M sodium phosphate buffer, pH 6.85, (7.0/3.0) to develop the choromatogram. The DMF solution was flash evaporated to near dryness and the reaction mixture was then taken up in dilute NaOH and the pH was adjusted to 2.5-3.0 to precipitate out the mixture. The precipitate was then centrifuged down and washed twice with diluted acetic acid in water. The precipitate was then centrifuged down and washed twice with diluted acetic acid in water. The precipitate was then redissolved in dilute NaOH and the pH adjusted to 7.0. This was applied to a reverse phase (C-18 silica) column 3.7 cm x 45 cm.

The product was eluted from the column with a solution of 0.1 M sodium phosphate buffer, pH 6.85/methanol (7.0/3.0). Fractions were collected and the fractions of pure di-L- $\alpha$ -serinyl chlorin $e_6$ were pooled. The methanol was flashed off and the product was precipitated at pH 2.5-3.0. The precipitate was centrifuged down and washed three times with dilute acetic acid in water. The product was lyophilized and produced a yield of 200 mg di-L- $\alpha$ -serinyl chlorin $e_6$.

Similarly, by utilizing other amino acids, peptides which further illustrate embodiments of, but do not limit the present invention, can be prepared using the procedure of the preceding examples:

Di, tri- (D,L)-serinyl chlorin $e_6$
Di, tri- (D,L)serinyl mesochlorin $e_6$
Di, tri- glycyl chlorin $e_6$
Di, tri- glycycl mesochlorin $e_6$
Di, tri- $\alpha$ -(D,L)-alanyl chlorin $e_6$
Di, tri- $\alpha$ -(D,L)-alanyl mesochlorin $e_6$
Di, tri- $\beta$ -alanyl chlorin $e_6$
Di, tri- $\beta$ -alanyl mesochlorin $e_6$
Di, tri- $\epsilon$ -amino-n-caproyl chlorin $e_6$
Di, tri- $\epsilon$- -amino-n-caproyl mesochlorin $e_6$
Di, tri- (D,L)-serinylbacteriochlorin $e_6$
Di, tri- glycylbacteriochlorin $e_6$
Di, tri- $\alpha$ -(D,L)-alanylbacteriochlorin $e_6$
Di, tri- $\beta$ -alanylbacteriochlorin $e_6$
Di, tri- $\epsilon$ -amino-n-caproylbacteriochlorin $e_6$
Di, tri- histidyl chlorin $e_6$
Di, tri- histidyl mesochlorin $e_6$
Di, tri- arginyl chlorin $e_6$
Di, tri- arginyl mesochlorin $e_6$
Di, tri- tyrosyl chlorin $e_6$
Di, tri- tyrosyl mesochlorin $e_6$
Di, tri- methionyl chlorin $e_6$
Di, tri- methionyl mesochlorin $e_6$
Di, tri- cysteinyl chlorin $e_6$
Di, tri- cysteinyl mesochlorin $e_6$
Di, tri- threoninyl chlorin $e_6$
Di, tri- threoninyl mesochlorin $e_6$
Di, tri- leucyl chlorin $e_6$
Di, tri- leucyl mesochlorin $e_6$
Threoninyl chlorin $e_6$
Tyrosyl chlorin $e_6$
Valyl chlorin $e_6$
Leucyl chlorin $e_6$
Isoleucyl chlorin $e_6$
Prolyl chlorin $e_6$
Methionyl chlorin $e_6$
Histidyl chlorin $e_6$
Arginyl chlorin $e_6$
Lysyl chlorin $e_6$
Glutaminyl chlorin $e_6$
4-hydroxyprolyl chlorin $e_6$

5-hydroxylysyl chlorin $e_6$
ϵ amino-n-caproyl chlorin $e_6$
γ aminobutanoyl chlorin $e_6$
3- methyl-histidyl chlorin $e_6$
Alanyl 2-acetyl-chlorin $e_6$
Valyl 2-acetyl-chlorin $e_6$
Leucyl 2-acetyl-chlorin $e_6$
Isoleucyl 2-acetyl-chlorin $e_6$
Prolyl 2-acetyl-chlorin $e_6$
Methionyl 2-acetyl-chlorin $e_6$
Glycyl 2-acetyl-chlorin $e_6$
Serinyl 2-acetyl-chlorin $e_6$
Threoninyl 2-acetyl-chlorin $e_6$
Cysteinyl 2-acetyl-chlorin $e_6$
Tyrosyl 2-acetyl-chlorin $e_6$
Asparginyl 2-acetyl-chlorin $e_6$
Lysyl 2-acetyl-chlorin $e_6$
Arginyl 2-acetyl-chlorin $e_6$
Histidyl 2-acetyl-chlorin $e_6$
Glutaminyl 2-acetyl-chlorin $e_6$
4-hydroxy-prolyl 2-acetyl-chlorin $e_6$
5-hydroxy lysyl 2-acetyl-chlorin $e_6$
ϵ-amino-n-caproyl 2-acetyl-chlorin $e_6$
γ-aminobutanoyl 2-acetyl-chlorin $e_6$
3-methyl histidyl 2-acetyl-chlorin $e_6$
β-alanyl 2-acetyl-chlorin $e_6$
Alanyl 2 formyl chlorin $e_6$
Valyl 2 formyl chlorin $e_6$
Leucyl 2 formyl chlorin $e_6$
Isoleucyl 2 formyl chlorin $e_6$
Prolyl 2 formyl chlorin $e_6$
Methionyl 2 formyl chlorin $e_6$
Glycyl 2 formyl chlorin $e_6$
Serinyl 2 formyl chlorin $e_6$
Threoninyl 2 formyl chlorin $e_6$
Cysteinyl 2 formyl chlorin $e_6$
Tyrosyl 2 formyl chlorin $e_6$
Asparginyl 2 formyl chlorin $e_6$
Lysyl 2 formyl chlorin $e_6$
Arginyl 2 formyl chlorin $e_6$
Histidyl 2 formyl chlorin $e_6$
Glutaminyl 2 formyl chlorin $e_6$
4-hydroxy-prolyl 2 formyl chlorin $e_6$
5-hydroxy lysyl 2 formyl chlorin $e_6$
ϵ-amino-n-caproyl 2 formyl chlorin $e_6$
γ-aminobutanoyl 2 formyl chlorin $e_6$
3-methyl histidyl 2 formyl chlorin $e_6$
β-alanyl 2 formyl chlorin $e_6$
Alanyl Deuterochlorin $e_6$
Valyl Deuterochlorin $e_6$
Leucyl Deuterochlorin $e_6$
Isoleucyl Deuterochlorin $e_6$
Prolyl Deuterochlorin $e_6$
Methionyl Deuterochlorin $e_6$
Glycyl Deuterochlorin $e_6$
Serinyl Deuterochlorin $e_6$
Threoninyl Deuterochlorin $e_6$
Cysteinyl Deuterochlorin $e_6$

Tyrosyl Deuterochlorin $\underline{e_6}$
Asparginyl Deuterochlorin $\underline{e_6}$
Lysyl Deuterochlorin $\underline{e_6}$
Arginyl Deuterochlorin $\underline{e_6}$
Histidyl Deuterochlorin $\underline{e_6}$
Glutaminyl Deuterochlorin $\underline{e_6}$
4-hydroxy-prolyl Deuterochlorin $\underline{e_6}$
5-hydroxy lysyl Deuterochlorin $\underline{e_6}$
ϵ-amino-n-caproyl Deuterochlorin $\underline{e_6}$
γ-aminobutanoyl Deuterochlorin $\underline{e_6}$
3-methyl histidyl Deuterochlorin $\underline{e_6}$
β-alanyl Deuterochlorin $\underline{e_6}$
Valyl mesochlorin $\underline{e_6}$
Leucyl mesochlorin $\underline{e_6}$
Isoleucyl mesochlorin $\underline{e_6}$
Prolyl mesochlorin $\underline{e_6}$
Methionyl mesochlorin $\underline{e_6}$
Serinyl mesochlorin $\underline{e_6}$
Threoninyl mesochlorin $\underline{e_6}$
Cysteinyl mesochlorin $\underline{e_6}$
Tyrosyl mesochlorin $\underline{e_6}$
Asparginyl mesochlorin $\underline{e_6}$
Lysyl mesochlorin $\underline{e_6}$
Arginyl mesochlorin $\underline{e_6}$
Histidyl mesochlorin $\underline{e_6}$
Glutaminyl mesochlorin $\underline{e_6}$
4-hydroxy-prolyl mesochlorin $\underline{e_6}$
5-hydroxy lysyl mesochlorin $\underline{e_6}$
γ-aminobutanoyl mesochlorin $\underline{e_6}$
3-methyl histidyl mesochlorin $\underline{e_6}$

Other amino acid derivatives of the tetrapyrroles can also be prepared. The following amino acids can also be used to prepare the di-,tri-, or where appropriate, the tetra-amino acid derivatives of the chlorins, porphyrins, or bacteriochlorins, employing the procedures of one of the aforementioned methods:
Piperidine-2-carboxylic acid;
Pyrrole-2-carboxylic acid;
Piperidine-2-propionic acid; and
Pyrrole-2-acetic acid.

Mixed amino acid derivatives of the tetrapyrroles can also be prepared. The various chlorin derivatives, porphyrin derivatives and bacteriochlorin derivatives can include any two or three of the following amino acids: Glycine, Serine, Threonine, Cysteine, Tyrosine, Asparagine, Glutamine, Lysine, Arginine, Histidine, α -Alanine, β -Alanine, Valine, Leucine, Isoleucine, Proline, α -Phenylalanine, β -Phenylalanine, Tryptophan, Methionine, ϵ -Amino-n-caproic acid, Piperidine-2-carboxylic acid, Pyrrole-2-carboxylic acid, Piperidine-2-propionic acid, Pyrrole-2-acetic acid.

The visible absorption spectrum in pyridine for all the amino acid derivatives of this invention are identical to that of the parent tetrapyrrole.

Physical characteristics of the compounds (relative polarity) is measured by a standard chromatographic system. The chromatographic data (Rf values) here measured on Baker silica gel-C18 thin layer chromatographic plates, the particle size of which is 20/μM, and the coating thickness of which is 200 μM. The solvent system for these chromatographic runs consisted of 75% methanol, and 25% 0.01 $\underline{M}$ potassium phosphate buffer, pH 6.85. The compounds were spotted and dried on the plate as the sodium salts, at approximately neutral pH and minimum salt concentrations. The Rf values for the various derivatives are tabulated in TABLE 1. Spectroscopic data are indicated in TABLE 2.

TABLE 1

| Rf VALUES | | |
|---|---|---|
| Compounds | Derivative | Rf |
| Chlorin $e_6$ | -- | .66 |
| Chlorin $e_6$ | di-L-α -serinyl | .78 |
| 2-formyl chlorin $e_6$ | ----- | 0.74 |
| 2-acetyl chlorin $e_6$ | ----- | 0.71 |
| Deutero chlorin $e_6$ | ----- | 0.79 |
| Mesochlorin $e_6$ | ----- | 0.69 |
| 2-formyl chlorin $e_6$ | Mono-L-serinyl | 0.87 |
| 2-acetyl chlorin $e_6$ | Mono-L-serinyl | 0.86 |
| Deuterochlorin $e_6$ | Mono-L-serinyl | 0.90 |
| Mesochlorin $e_6$ | Mono-L-serinyl | 0.73 |
| Chlorin $e_6$ | Mono-L-asparaginyl | 0.72 |
| Chlorin $e_6$ | Mono-L-cysteinyl | 0.93 |
| Chlorin $e_6$ | Mono-L-serinyl | 0.72 |

TABLE II

Spectroscopic Absorption Data

Solvent in all cases is p-dioxane.

| Compounds | Absorption Maxima (nm) in Visible Region | Soret Band nM |
|---|---|---|
| Mesochlorin $e_6$ | 651 | 399 |
| 2-acetyl-chlorin $e_6$ | 712,683 | 410 |
| 2-formyl-chlorin $e_6$ | 687 | 412 |
| Deuterochlorin $e_6$ | 653 | 398 |
| Chlorin $e_6$ | 666 | 402 |

Absorption data for the amino acid conjugates is identical to the parent chlorins.

The following protocols describe the procedure for the use of the therapeutic compositions of the present invention in the treatment of rat tumors.

EXAMPLE 13

The photodynamic therapy experiments have been carried out on Buffalo rats, using the transplantable tumor, Morris Hepatoma 7777. The tumors were transplanted subcutaneously on the outside of the thigh. During treatment, the tumors ranges in size between 1 and 2.5 cm in diameter.

The general treatment regime is as follows. The rats are injected with a solution of the chlorin prepared as follows: 20 mg of the sodium salt of the chlorin was dissolved in 1 ml of 0.9% NaCl. The chlorin solution was then injected intravenously through the external jugular while the rat was anesthetized with ether. The volume of solution injected was calculated based upon the weight of the animal and the dosage, on a weight to weight basis, for the particular experiment. A specified time interval was then allowed to elapse before light treatment was instigated.

Light treatment of the rats was without anesthesia. The rats were restrained, the hair removed in the treatment area and treated with laser light from a Cooper Aurora argon pumped, tunable dye laser.

The laser was equipped with a fiber optic light delivery system coupled to a microlens system

developed by Dr. Daniel Doiron, D.R.D. Consulting, Santa Barbara, California.

The lens disperses the laser beam, providing a circular distribution of light with homogenous light intensity throughout the area of the incident light beam. The wavelength of light was adjusted using a Hartridge reversion spectroscope. The light intensity was determined using a Yellow Springs Instrument, Model 65A, radiometer.

The micro lens was positioned at such a distance from the skin of the animal so as to provide an illumination diameter of 1.5cm, and the light flux was varied by control of the laser output.

Subsequent to illumination, the animal was returned to its cage and, 24 hours later, it was treated intravenously in the external jugular vein with 14 mg of Evans Blue dye, dissolved in 250 µl of 0.9% NaCl. Two hours after injection, the rat was sacrificed and the tumor cross-sectioned. The extent of tumor necrosis was assessed by the lack of dye uptake [(1)] , and the depth of the necrotic cross section of the tumor was recorded in millimeters.

Table III summarizes the effects of these drugs on tumors. The conditions described result in measurable and significant damage to the tumors.

In all cases except where noted, tissue damage occurred selectively to the tumor tissue as assayed by the Evans Blue method, even though, in nearly all cases, normal skin overlayed the tumor and the treatment area overlapped significant areas of normal muscle tissue.

The photodynamic therapy data is presented in tabular form. Column No. 2 is the total light dose administered in terms of Joules per square centimeter. Column No. 3 is the dose of chlorin administered in terms of mg of drug per kilogram of rat body weight. Column No. 4 is the time lapse between administration of drug and treatment with laser light. Column No. 5 is the wavelength of treatment light in nanometers. Column No. 6 is the intensity of the treatment light in milliwatts per square centimeter. In Column No. 7, $\bar{x}$ is the mean depth of necrosis in millimeters of the tumor tissue, i.e., the distance from the necrotic top of the tumor next to the skin to the necrotic edge of the tumor most distant from the skin.

S.D. is the standard deviation of $\bar{x}$.

(N) is the number of tumors or legs involved in the experiment.

Column No. 8 is the range of depth of necrosis in millimeters within the group.

(1) M.C. Berenbaum, Br. J. Cancer , 45: 571(1982)

TABLE III

| Tumor | Joules/ $cm^2$ | Drug dose mg/kg | Time in hrs btwn drugs & light | Wave lnth nm | Intensity mW/$cm^2$ | $\bar{x}$ s.d. | (n) | Range mm |
|---|---|---|---|---|---|---|---|---|
| | | | | Mono Glycyl chlorin $e_6$ | | | | |
| 777 | 20 | 20 | 24 | 665 | 100 | 3.9±3.0 | (5) | 2-9 * |
| | | | | Mono-L-α-alanyl chlorin $e_6$ | | | | |
| 777 | 20 | 20 | 24 | 665 | 100 | 3.8±1.8 | (2) | 2.5-5 |
| | | | | Mono-L-α-serinyl chlorin $e_6$ | | | | |
| 777 | 20 | 20 | 24 | 665 | 100 | 6.3±2.7 | (6) | 3-10 |

of 8 tumors showed no necrosis due to drug and light.

EXAMPLE 14

The treatment and evaluation procedure is as follows:

DBA/2 Ha Ros-d + Ha mice with SmT-F transplanted tumors either in the exterior part of the hind leg or

the side of the mouse were injected intravenously via the external jugular or the intraperitoneally with the photosensitizing drug. At the specified time after injection, the area over the tumor was shaved and the light treatment begun.

Light from a Copper Aurora argon pumped tunable dye laser was administered via a micro lens system (developed by Dr. Daniel Doiron, D.R.D. Consulting, Santa Barbara, California) coupled through a quartz fiber to the laser, the optical properties of the lens are such that the light exits the lens in a circular pattern with homogenous intensity throughout the lighted area. The diameter of the lighted area is a function of the distance from the lens.

The light intensity was measured with a Yellow Springs Instrument Model 65 A Radiometer at the point of treatment. A 1.5 cm diameter circle of the animal's skin, centered as closely as possible over the tumor, was irradiated in all the experiments. The intensity, wavelength, and dosage of light is included in the data for individual groups of animals. Wavelengths are adjusted, using a Hartridge reversion spectroscope to within 1 nm of the stated value.

Twenty four hours after light treatment, each mouse received 5 mg of Evans Blue Dye intravenously [(1)].After an additional two hours, the mice were sacrificed and the tumors were sectioned vertically through the center of the light treated area. Unaffected tumor was stained blue as was unaffected normal tissue. Necrotic or affected areas were white or red in appearance. Measurements on both the whole tumors and affected areas of the tumors were made vertically and horizontally with calipers to the nearest one half millimeter. The results of representative compounds are depicted in the following tables:

TABLE IV - MOUSE DATA

| COMPOUND USED | Mono-L-serinyl mesochlorin e6 | | | | |
|---|---|---|---|---|---|
| ANIMAL GROUP NO. | 78 | 78 | 78 | 78 | 78 |
| DATE EXPERIMENT STARTED | -- | -- | -- | -- | -- |
| MOUSE NO. | 1 | 2 | 3 | 4 | 5 |
| SEX OF MOUSE | m | m | m | m | m |
| WT. OF MOUSE (gms) | 23.3 | 25.8 | 21.0 | 22.8 | 26.4 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 651 | 651 | 651 | 651 | 651 |
| DATE ANIMAL INJECTED WITH DRUG | -- | -- | -- | -- | -- |
| LENGTH OF TUMOR ON INJECTION DATE (cm) | 1.00 | 0.90 | 0.75 | 0.55 | 1.05 |
| WIDTH OF TUMOR ON INJECTION DATE (cm) | 0.60 | 0.55 | 0.65 | 0.45 | 0.45 |
| DEPTH OF TUMOR ON INJECTION DATE (cm) | 0.45 | 0.30 | 0.30 | 0.20 | 0.25 |
| DATE ANIMAL SACRIFICED | -- | -- | -- | -- | -- |
| LENGTH OF TUMOR ON SACRIFICE DATE (cm) | 1.30 | 1.30 | 0.30 | 0.90 | 1.30 |
| WIDTH OF TUMOR ON SACRIFICE DATE (cm) | 1.20 | 1.00 | 0.80 | 0.70 | 0.85 |
| DEPTH OF TUMOR ON SACRIFICE DATE (cm) | 0.65 | 0.70 | 0.65 | 0.60 | 0.60 |
| LENGTH OF EFFECT UPON TUMOR ON SACRIFICE DATE (cm) | 0.00 | 0.80 | 0.10 | 0.60 | 0.00 |
| WIDTH OF EFFECT UPON TUMOR ON SACRIFICE DATE (cm) | 0.00 | 0.40 | 0.10 | 0.60 | 0.00 |
| DEPTH OF EFFECT UPON TUMOR ON SACRIFICE DATE (cm) | 0.00 | 0.30 | 0.10 | 0.20 | 0.00 |
| COMMENTS AS RESULT OF TUMOR ASSESSMENT | no effect | red skin effect 0.9x0.9 cm | | | red skin effect 0.6x0.6 cm; no effect on the tumor |

Table IV

| Compound | Mono-L-serinyl mesochlorin e6 | | | | |
|---|---|---|---|---|---|
| GROUP NO. | 78 | ← | ← | ← | ← |
| START DATE | | | | | |
| MOUSE NO. | 1 | 2 | 3 | 4 | 5 |
| SEX | m. | ← | ← | ← | ← |
| MOUSE WHT | 23.3 | 25.8 | 21.0 | 22.8 | 26.4. |
| DOSE | 100.0 | ← | ← | ← | ← |
| METHOD | iv | ← | ← | ← | ← |
| TIME | 24.0 | ← | ← | ← | ← |
| TUMOR TYPE | SMT-F | ← | ← | ← | ← |
| TUMOR POSIT | r leg | ← | ← | ← | ← |
| LIGHT INTEN | 200.0 | ← | ← | ← | ← |
| LIGHT DOSE | 300.0 | ← | ← | ← | ← |
| WAVE LENGTH | 651 | ← | ← | ← | ← |
| INJECT DATE | | | | | |
| LENGTH 1 | 1.00 | 0.90 | 0.75 | 0.55 | 1.05 |
| WIDTH 1 | 0.60 | 0.55 | 0.65 | 0.45 | 0.45 |
| DEPTH 1 | 0.45 | 0.30 | 0.30 | 0.20 | 0.25 |
| KILL DATE | | | | | |
| LENGTH 2 | 1.30 | 1.30 | 0.30 | 0.90 | 1.30 |
| WIDTH 2 | 1.20 | 1.00 | 0.80 | 0.70 | 0.85 |
| DEPTH 2 | 0.65 | 0.70 | 0.65 | 0.60 | 0.60 |
| LENGTH 3 | 0.00 | 0.80 | 0.10 | 0.60 | 0.00 |
| WIDTH 3 | 0.00 | 0.40 | 0.10 | 0.60 | 0.00 |
| DEPTH 3 | 0.00 | 0.30 | 0.10 | 0.20 | 0.00 |
| COMMENTS | no effect | red skin effect 0.9x0.9cm | | | red skin effect 0.6x0.6cm. no effect on the tumor |

TABLE V - MOUSE DATA

| COMPOUND USED | Mono-L-serinyl 2-acetyl chlorin e6 | |
|---|---|---|
| ANIMAL GROUP NO. | 81 | 81 |
| DATE EXPERIMENT STARTED | -- | -- |
| MOUSE NO. | 1 | 2 |
| SEX OF MOUSE | m | m |
| WT. OF MOUSE (gms) | 26.5 | 21.0 |
| DRUG DOSE (mg/kg) | 100.0 | 100.0 |
| METHOD OF DRUG INTRODUCTION | iv | iv |
| TIME BETW. DRUG INTRODUCTION + LIGHT TREATMENT (hrs) | 24.0 | 24.0 |
| TUMOR TYPE | SMT-F | SMT-F |
| POSITION OF TUMOR ON ANIMAL | r leg | r leg |
| LIGHT TREATMENT INTENSITY ($mW/cm^2$) | 200.0 | 200.0 |
| LIGHT DOSE ($J/cm^2$) | 300.0 | 300.0 |
| WAVE LENGTH USED TO TREAT TUMOR (nm) | 680 | 680 |
| DATE ANIMAL INJECTED WITH DRUG | -- | -- |
| LENGTH OF TUMOR ON INJECTION DATE (cm) | 0.80 | 0.80 |
| WIDTH OF TUMOR ON INJECTION DATE (cm) | 0.45 | 0.60 |
| DEPTH OF TUMOR ON INJECTION DATE (cm) | 0.40 | 0.40 |
| DATE ANIMAL SACRIFICED | -- | -- |
| LENGTH OF TUMOR ON SACRIFICE DATE (cm) | 1.20 | 0.90 |
| WIDTH OF TUMOR ON SACRIFICE DATE (cm) | 0.90 | 0.70 |
| DEPTH OF TUMOR ON SACRIFICE DATE (cm) | 0.60 | 0.40 |
| LENGTH OF EFFECT UPON TUMOR ON SACRIFICE DATE (cm) | 0.00 | 0.00 |
| WIDTH OF EFFECT UPON TUMOR ON SACRIFICE DATE (cm) | 0.00 | 0.00 |
| DEPTH OF EFFECT UPON TUMOR ON SACRIFICE DATE (cm) | 0.00 | 0.00 |
| COMMENTS AS RESULT OF TUMOR ASSESSMENT | no effect | no effect |

TABLE VI - MOUSE DATA

| COMPOUND USED | Mono-L-serinyl deuterochlorin e6 | | | | |
|---|---|---|---|---|---|
| ANIMAL GROUP NO. | 82 | 82 | 82 | 82 | 82 |
| DATE EXPERIMENT STARTED | -- | -- | -- | -- | -- |
| MOUSE NO. | 1 | 2 | 3 | 4 | 5 |
| SEX OF MOUSE | m | m | m | m | m |
| WT. OF MOUSE (gms) | 25.7 | 23.2 | 21.3 | 20.5 | 24.4 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 655 | 655 | 655 | 655 | 655 |
| DATE ANIMAL INJECTED WITH DRUG | -- | -- | -- | -- | -- |
| LENGTH OF TUMOR ON INJECTION DATE (cm) | 1.40 | 1.75 | 1.90 | 1.45 | 1.35 |
| WIDTH OF TUMOR ON INJECTION DATE (cm) | 1.15 | 1.10 | 0.65 | 1.05 | 0.85 |
| DEPTH OF TUMOR ON INJECTION DATE (cm) | 0.75 | 1.00 | 0.20 | 0.90 | 0.65 |
| DATE ANIMAL SACRIFICED | -- | -- | -- | -- | -- |
| LENGTH OF TUMOR ON SACRIFICE DATE (cm) | 1.70 | 1.80 | 2.20 | 1.75 | 1.50 |
| WIDTH OF TUMOR ON SACRIFICE DATE (cm) | 0.80 | 1.15 | 1.00 | 1.25 | 0.85 |
| DEPTH OF TUMOR ON SACRIFICE DATE (cm) | 0.60 | 0.60 | 0.80 | 0.50 | 0.65 |
| LENGTH OF EFFECT UPON TUMOR ON SACRIFICE DATE (cm) | 0.30 | 0.40 | 0.40 | 1.00 | 0.00 |
| WIDTH OF EFFECT UPON TUMOR ON SACRIFICE DATE (cm) | 0.35 | 0.40 | 0.40 | 1.15 | 0.00 |
| DEPTH OF EFFECT UPON TUMOR ON SACRIFICE DATE (cm) | 0.15 | 0.20 | 0.20 | 0.20 | 0.00 |
| COMMENTS AS RESULT OF TUMOR ASSESSMENT | | | | | no effect |

TABLE VII - MOUSE DATA

| COMPOUND USED | Mono-L-asparaginyl chlorin e6 | | | | |
|---|---|---|---|---|---|
| ANIMAL GROUP NO. | 83 | 83 | 83 | 83 | 83 |
| DATE EXPERIMENT STARTED | -- | -- | -- | -- | -- |
| MOUSE NO. | 1 | 2 | 3 | 4 | 5 |
| SEX OF MOUSE | m | m | m | m | m |
| WT. OF MOUSE (gms) | 25.4 | 25.0 | 25.8 | 24.6 | 24.1 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 665 | 665 | 665 | 665 | 665 |
| DATE ANIMAL INJECTED WITH DRUG | -- | -- | -- | -- | -- |
| LENGTH OF TUMOR ON INJECTION DATE (cm) | 1.30 | 1.20 | 1.30 | 1.25 | 1.00 |
| WIDTH OF TUMOR ON INJECTION DATE (cm) | 0.90 | 0.90 | 1.05 | 0.75 | 0.70 |
| DEPTH OF TUMOR ON INJECTION DATE (cm) | 0.60 | 0.55 | 0.60 | 0.60 | 0.50 |
| DATE ANIMAL SACRIFICED | -- | -- | -- | -- | -- |
| LENGTH OF TUMOR ON SACRIFICE DATE (cm) | 1.05 | 1.40 | 1.60 | 1.60 | 1.30 |
| WIDTH OF TUMOR ON SACRIFICE DATE (cm) | 0.90 | 0.85 | 0.80 | 0.75 | 0.90 |
| DEPTH OF TUMOR ON SACRIFICE DATE (cm) | 0.65 | 0.65 | 0.60 | 0.65 | 0.60 |
| LENGTH OF EFFECT UPON TUMOR ON SACRIFICE DATE (cm) | 1.05 | 1.40 | 1.10 | 1.60 | 1.05 |
| WIDTH OF EFFECT UPON TUMOR ON SACRIFICE DATE (cm) | 0.90 | 0.85 | 0.50 | 0.75 | 0.60 |
| DEPTH OF EFFECT UPON TUMOR ON SACRIFICE DATE (cm) | 0.50 | 0.60 | 0.35 | 0.65 | 0.45 |
| COMMENTS AS RESULT OF TUMOR ASSESSMENT | skin effect 0.65x0.60 cm | skin effect 0.8x.0.9 cm | | skin effect 0.95x0.95 cm muscle damage | skin effect 0.5x0.5 cm |

TABLE VIII - MOUSE DATA

| COMPOUND USED | Mono-L-serinyl-2-formyl chlorin e6 | | | |
|---|---|---|---|---|
| ANIMAL GROUP NO. | 85 | 85 | 85 | 85 |
| DATE EXPERIMENT STARTED | -- | -- | -- | -- |
| MOUSE NO. | 1 | 2 | 3 | 4 |
| SEX OF MOUSE | m | m | m | m |
| WT. OF MOUSE (gms) | 26.0 | 20.5 | 20.2 | 28.8 |
| DRUG DOSE (mg/kg) | 100.0 | 100.0 | 100.0 | 100.0 |
| METHOD OF DRUG INTRODUCTION | iv | iv | iv | iv |
| TIME BETW. DRUG INTRODUCTION + LIGHT TREATMENT (hrs) | 24.0 | 24.0 | 24.0 | 24.0 |
| TUMOR TYPE | SMT-F | SMT-F | SMT-F | SMT-F |
| POSITION OF TUMOR ON ANIMAL | r leg | r leg | r leg | r leg |
| LIGHT TREATMENT INTENSITY ($mW/cm^2$) | 200.0 | 200.0 | 200.0 | 200.0 |
| LIGHT DOSE ($J/cm^2$) | 300.0 | 300.0 | 300.0 | 300.0 |
| WAVE LENGTH USED TO TREAT TUMOR (nm) | 690 | 690 | 690 | 690 |
| DATE ANIMAL INJECTED WITH DRUG | -- | -- | -- | -- |
| LENGTH OF TUMOR ON INJECTION DATE (cm) | 1.60 | 1.70 | 1.80 | 1.60 |
| WIDTH OF TUMOR ON INJECTION DATE (cm) | 1.00 | 1.10 | 1.20 | 1.00 |
| DEPTH OF TUMOR ON INJECTION DATE (cm) | 0.70 | 0.75 | 0.60 | 0.70 |
| DATE ANIMAL SACRIFICED | -- | -- | -- | -- |
| LENGTH OF TUMOR ON SACRIFICE DATE (cm) | 1.60 | 1.60 | 1.00 | 1.70 |
| WIDTH OF TUMOR ON SACRIFICE DATE (cm) | 1.05 | 1.10 | 1.30 | 1.10 |
| DEPTH OF TUMOR ON SACRIFICE DATE (cm) | 0.75 | 0.80 | 0.80 | 0.80 |
| LENGTH OF EFFECT UPON TUMOR ON SACRIFICE DATE (cm) | 0.40 | 1.30 | 0.90 | 0.00 |
| WIDTH OF EFFECT UPON TUMOR ON SACRIFICE DATE (cm) | 0.30 | 0.60 | 0.80 | 0.00 |
| DEPTH OF EFFECT UPON TUMOR ON SACRIFICE DATE (cm) | 0.15 | 0.25 | 0.30 | 0.00 |
| COMMENTS AS RESULT OF TUMOR ASSESSMENT | | | | no effect |

TABLE IX - MOUSE DATA

| COMPOUND USED | Mono-L-cysteinyl chlorin e6 | | | | |
|---|---|---|---|---|---|
| ANIMAL GROUP NO. | 86 | 86 | 86 | 86 | 86 |
| DATE EXPERIMENT STARTED | -- | -- | -- | -- | -- |
| MOUSE NO. | 1 | 2 | 3 | 4 | 5 |
| SEX OF MOUSE | m | m | m | m | m |
| WT. OF MOUSE (gms) | 26.0 | 26.2 | 27.1 | 22.2 | 26.0 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 200.0 | 200.0 | 200.0 | 200.0 | 200.0 |
| LIGHT DOSE ($J/cm^2$) | 300.0 | 300.0 | 300.0 | 300.0 | 300.0 |
| WAVE LENGTH USED TO TREAT TUMOR (nm) | 665 | 665 | 665 | 665 | 665 |
| DATE ANIMAL INJECTED WITH DRUG | -- | -- | -- | -- | -- |
| LENGTH OF TUMOR ON INJECTION DATE (cm) | 1.45 | 1.60 | 2.05 | 0.90 | 1.80 |
| WIDTH OF TUMOR ON INJECTION DATE (cm) | 1.00 | 1.20 | 1.60 | 0.85 | 1.30 |
| DEPTH OF TUMOR ON INJECTION DATE (cm) | 0.75 | 0.65 | 0.90 | 0.60 | 0.70 |
| DATE ANIMAL SACRIFICED | -- | -- | -- | -- | -- |
| LENGTH OF TUMOR ON SACRIFICE DATE (cm) | 1.40 | 1.80 | 1.80 | 1.00 | 2.00 |
| WIDTH OF TUMOR ON SACRIFICE DATE (cm) | 1.00 | 1.05 | 1.40 | 1.10 | 1.30 |
| DEPTH OF TUMOR ON SACRIFICE DATE (cm) | 0.80 | 0.70 | 0.80 | 0.75 | 0.80 |
| LENGTH OF EFFECT UPON TUMOR ON SACRIFICE DATE (cm) | 1.40 | 1.60 | 1.60 | 1.00 | 1.85 |
| WIDTH OF EFFECT UPON TUMOR ON SACRIFICE DATE (cm) | 1.00 | 1.05 | 1.10 | 1.10 | 1.20 |
| DEPTH OF EFFECT UPON TUMOR ON SACRIFICE DATE (cm) | 0.80 | 0.45 | 0.60 | 0.70 | 0.75 |
| COMMENTS AS RESULT OF TUMOR ASSESSMENT | red skin 1.3x1.0 cm some muscle damage | skin effect 1.6x1.2 cm some muscle damage | skin effect 1.4x1.4 cm | skin effect 0.9x0.9 cm some muscle damage | skin effect 1.5x1.4 cm |

The results of Table IV - IX are summarized in Table X.

TABLE X

| compound | drug dose mg/kg | method of injection | time in hrs between drug dose & light | tumor type | light intensity mW/cm² | light dose J/cm² | Wave-length nm | (n) | $\bar{X} \pm$ s.d. (cm) | range cm |
|---|---|---|---|---|---|---|---|---|---|---|
| mono-l-asparaginyl chlorin e6 | 100 | iv | 24 | SMT-F | 200 | 300 | 665 | (5) | 0.51±0.12 | 0.35 0 |
| mono-l-cysteinyl chlorin e6 | 100 | iv | 24 | SMT F | 200 | 300 | 665 | (5) | 0.66±0.14 | 0.45 0 |
| mono-l serinyl 2 acetyl chlorin e6 | 100 | iv | 24 | SMT F | 200 | 300 | 680 | (2) | 0.00 | 0 |
| mono-l-serinyl 2 formyl chlorin e6 | 100 | iv | 24 | SMT-F | 200 | 300 | 690 | (4) | 0.18±0.13 | 0.00 0 |
| mono-l-serinyl deuterochlorin e6 | 100 | iv | 24 | SMT F | 200 | 300 | 655 | (5) | 0.15±0.09 | 0.00 0 |
| mono-l-serinyl mesochlorin e6 | 100 | iv | 24 | SMT F | 200 | 300 | 651 | (5) | 0.12±0.13 | 0.00 0 |

TABLE XI - MOUSE DATA

| COMPOUND USED | Mono-L- serinyl chlorin e6 | | | | | |
|---|---|---|---|---|---|---|
| ANIMAL GROUP NO. | 49 | 49 | 49 | 49 | 49 | 49 |
| DATE EXPERIMENT STARTED | -- | -- | -- | -- | -- | -- |
| MOUSE NO. | 1 | 2 | 3 | 4 | 5 | 6 |
| SEX OF MOUSE | m | f | f | f | f | f |
| WT. OF MOUSE (gms) | 24.8 | 22.1 | 20.2 | 16.4 | 20.7 | 22.7 |
| DRUG DOSE (mg/kg) | 100.0 | 100.0 | 100.0 | 100.0 | 100.0 | 100.00 |
| METHOD OF DRUG INTRODUCTION | iv | iv | iv | iv | iv | iv |
| TIME BETW. DRUG INTRODUCTION + LIGHT TREATMENT (hrs) | 24.0 | 24.0 | 24.0 | 24.0 | 24.0 | 24.0 |
| TUMOR TYPE | SMT-F | SMT-F | SMT-F | SMT-F | SMT-F | SMT-F |
| POSITION OF TUMOR ON ANIMAL | r leg | r leg | r leg | r leg | r leg | r leg |
| LIGHT TREATMENT INTENSITY ($mW/cm^2$) | 75.0 | 75.0 | 75.0 | 75.0 | 75.0 | 75.0 |
| LIGHT DOSE ($J/cm^2$) | 20.0 | 20.0 | 20.0 | 20.0 | 20.0 | 20.0 |
| WAVE LENGTH USED TO TREAT TUMOR (nm) | 665 | 665 | 665 | 665 | 665 | 665 |
| DATE ANIMAL INJECTED WITH DRUG | -- | -- | -- | -- | -- | -- |
| LENGTH OF TUMOR ON INJECTION DATE (cm) | 0.00 | 1.40 | 2.00 | 1.50 | 1.60 | 1.60 |
| WIDTH OF TUMOR ON INJECTION DATE (cm) | 0.00 | 0.80 | 1.00 | 0.90 | 1.05 | 0.90 |
| DEPTH OF TUMOR ON INJECTION DATE (cm) | 0.00 | 0.65 | 0.60 | 0.60 | 0.65 | 0.70 |
| DATE ANIMAL SACRIFICED | -- | -- | -- | -- | -- | -- |
| LENGTH OF TUMOR ON SACRIFICE DATE (cm) | 0.00 | 1.60 | 2.20 | 1.90 | 1.20 | 1.70 |
| WIDTH OF TUMOR ON SACRIFICE DATE (cm) | 0.00 | 0.85 | 1.15 | 1.15 | 1.05 | 1.05 |
| DEPTH OF TUMOR ON SACRIFICE DATE (cm) | 0.00 | 0.45 | 0.65 | 0.60 | 0.80 | 0.80 |
| LENGTH OF EFFECT UPON TUMOR ON SACRIFICE DATE (cm) | 0.00 | 1.60 | 1.20 | 1.50 | 1.20 | 1.50 |
| WIDTH OF EFFECT UPON TUMOR ON SACRIFICE DATE (cm) | 0.00 | 0.85 | 1.00 | 1.20 | 1.05 | 0.95 |
| DEPTH OF EFFECT UPON TUMOR ON SACRIFICE DATE (cm) | 0.00 | 0.45 | 0.50 | 0.50 | 0.50 | 0.50 |
| COMMENTS AS RESULT OF TUMOR ASSESSMENT | died of the ether | leg swollen skin pink over the treat-ment area, top 2/3 of tumor red | leg swollen skin pink over the treat-ment area, tumor red on top | leg swollen skin pink over the treat-ment site, tumor red on top | leg swollen skin pink over the treat-ment site, top 2/3 of tumor red | leg swollen skin pink over the treat-ment area, top 2/3 of tumor red |

TABLE XI - MOUSE DATA (cont'd)

| COMPOUND USED | Mono-L-.-serinyl chlorin e6 | | | | |
|---|---|---|---|---|---|
| ANIMAL GROUP NO. | 49 | 49 | 49 | 49 | 49 |
| DATE EXPERIMENT STARTED | -- | -- | -- | -- | -- |
| MOUSE NO. | 7 | 8 | 9 | 10 | 11 |
| SEX OF MOUSE | f | f | f | f | m |
| WT. OF MOUSE (gms) | 20.9 | 19.4 | 20.6 | 20.2 | 19.6 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 0.0 |
| TUMOR TYPE | SMT-F | SMT-F | SMT-F | SMT-F | 0.0 |
| POSITION OF TUMOR ON ANIMAL | r leg | r leg | r leg | r leg | 0.0 |
| LIGHT TREATMENT INTENSITY ($mW/cm^2$) | 75.0 | 75.0 | 75.0 | 75.0 | 0.0 |
| LIGHT DOSE ($J/cm^2$) | 20.0 | 20.0 | 20.0 | 20.0 | 0.0 |
| WAVE LENGTH USED TO TREAT TUMOR (nm) | 665 | 665 | 665 | 665 | 0.0 |
| DATE ANIMAL INJECTED WITH DRUG | -- | -- | -- | -- | -- |
| LENGTH OF TUMOR ON INJECTION DATE (cm) | 1.50 | 0.90 | 1.70 | 1.30 | 0.00 |
| WIDTH OF TUMOR ON INJECTION DATE (cm) | 1.25 | 0.75 | 0.95 | 0.90 | 0.00 |
| DEPTH OF TUMOR ON INJECTION DATE (cm) | 0.70 | 0.70 | 0.65 | 0.60 | 0.00 |
| DATE ANIMAL SACRIFICED | -- | -- | -- | -- | -- |
| LENGTH OF TUMOR ON SACRIFICE DATE (cm) | 1.90 | 1.45 | 1.70 | 1.70 | 0.00 |
| WIDTH OF TUMOR ON SACRIFICE DATE (cm) | 1.35 | 0.95 | 1.00 | 1.10 | 0.00 |
| DEPTH OF TUMOR ON SACRIFICE DATE (cm) | 0.85 | 0.80 | 0.95 | 0.85 | 0.00 |
| LENGTH OF EFFECT UPON TUMOR ON SACRIFICE DATE (cm) | 1.50 | 1.30 | 1.40 | 1.10 | 0.00 |
| WIDTH OF EFFECT UPON TUMOR ON SACRIFICE DATE (cm) | 1.00 | 0.95 | 0.90 | 0.95 | 0.00 |
| DEPTH OF EFFECT UPON TUMOR ON SACRIFICE DATE (cm) | 0.70 | 0.60 | 0.60 | 0.45 | 0.00 |
| COMMENTS AS RESULT OF TUMOR ASSESSMENT | leg swollen, skin pink over treatment site, top 1/2 of tumor red | leg swollen, skin pink over treatment site, top 2/3 of tumor red | leg swollen, skin pink over treatment site, top 2/3 of tumor red | leg swollen, skin pink over treatment site, top 1/2 of tumor red | died of ether after the injection |

TABLE XII - MOUSE DATA

| COMPOUND USED | Mono glycyl chlorin e6 | | | | |
|---|---|---|---|---|---|
| ANIMAL GROUP NO. | 47 | 47 | 47 | 47 | 47 |
| DATE EXPERIMENT STARTED | -- | -- | -- | -- | -- |
| MOUSE NO. | 1 | 2 | 3 | 4 | 5 |
| SEX OF MOUSE | f | f | f | f | f |
| WT. OF MOUSE (gms) | 20.4 | 18.7 | 21.3 | 19.6 | 18.4 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 75.0 | 75.0 | 75.0 | 75.0 | 75.0 |
| LIGHT DOSE ($J/cm^2$) | 20.0 | 20.0 | 20.0 | 20.0 | 20.0 |
| WAVE LENGTH USED TO TREAT TUMOR (nm) | 665 | 665 | 665 | 665 | 665 |
| DATE ANIMAL INJECTED WITH DRUG | -- | -- | -- | -- | -- |
| LENGTH OF TUMOR ON INJECTION DATE (cm) | 1.60 | 1.10 | 1.80 | 1.85 | 1.35 |
| WIDTH OF TUMOR ON INJECTION DATE (cm) | 1.00 | 0.95 | 1.10 | 1.50 | 1.05 |
| DEPTH OF TUMOR ON INJECTION DATE (cm) | 0.80 | 0.65 | 0.60 | 0.85 | 0.65 |
| DATE ANIMAL SACRIFICED | -- | -- | -- | -- | -- |
| LENGTH OF TUMOR ON SACRIFICE DATE (cm) | 1.20 | 0.00 | 1.65 | 1.40 | 1.25 |
| WIDTH OF TUMOR ON SACRIFICE DATE (cm) | 0.90 | 0.00 | 1.40 | 1.00 | 0.95 |
| DEPTH OF TUMOR ON SACRIFICE DATE (cm) | 0.90 | 0.00 | 1.00 | 0.90 | 0.65 |
| LENGTH OF EFFECT UPON TUMOR ON SACRIFICE DATE (cm) | 0.70 | 0.00 | 0.40 | 0.30 | 0.70 |
| WIDTH OF EFFECT UPON TUMOR ON SACRIFICE DATE (cm) | 0.60 | 0.00 | 0.60 | 0.60 | 0.80 |
| DEPTH OF EFFECT UPON TUMOR ON SACRIFICE DATE (cm) | 0.30 | 0.00 | 0.20 | 0.20 | 0.25 |
| COMMENTS AS RESULT OF TUMOR ASSESSMENT | | died from the dye injection; cannot read | | | |

TABLE XII - MOUSE DATA (cont'd)

| COMPOUND USED | Mono glycyl chlorin e6 | | | | |
|---|---|---|---|---|---|
| ANIMAL GROUP NO. | 47 | 47 | 47 | 47 | 47 |
| DATE EXPERIMENT STARTED | -- | -- | -- | -- | -- |
| MOUSE NO. | 6 | 7 | 8 | 9 | 10 |
| SEX OF MOUSE | f | f | f | f | f |
| WT. OF MOUSE (gms) | 19.6 | 19.1 | 20.1 | 19.8 | 19.6 |
| DRUG DOSE (mg/kg) | 100.0 | 100.0 | 100.0 | 100.0 | 100.0 |
| METHOD OF DRUG INTRODUCTION | iv | iv | iv | iv | iv |
| TIME BETW. DRUG INTRODUCTION + LIGHT TREATMENT (hrs) | 24.0 | 24.0 | 24.0 | 24.0 | 24.0 |
| TUMOR TYPE | SMT-F | SMT-F | SMT-F | SMT-F | SMT-F |
| POSITION OF TUMOR ON ANIMAL | r leg | r leg | r leg | r leg | r leg |
| LIGHT TREATMENT INTENSITY ($mW/cm^2$) | 75.0 | 75.0 | 75.0 | 75.0 | 75.0 |
| LIGHT DOSE ($J/cm^2$) | 20.0 | 20.0 | 20.0 | 20.0 | 20.0 |
| WAVE LENGTH USED TO TREAT TUMOR (nm) | 665 | 665 | 665 | 665 | 665 |
| DATE ANIMAL INJECTED WITH DRUG | -- | -- | -- | -- | -- |
| LENGTH OF TUMOR ON INJECTION DATE (cm) | 1.30 | 1.35 | 1.35 | 1.35 | 1.30 |
| WIDTH OF TUMOR ON INJECTION DATE (cm) | 0.95 | 1.00 | 0.90 | 1.05 | 0.90 |
| DEPTH OF TUMOR ON INJECTION DATE (cm) | 0.70 | 0.80 | 0.60 | 0.60 | 0.50 |
| DATE ANIMAL SACRIFICED | -- | -- | -- | -- | -- |
| LENGTH OF TUMOR ON SACRIFICE DATE (cm) | 1.05 | 1.35 | 1.40 | 1.35 | 1.20 |
| WIDTH OF TUMOR ON SACRIFICE DATE (cm) | 1.00 | 1.00 | 1.10 | 1.00 | 1.10 |
| DEPTH OF TUMOR ON SACRIFICE DATE (cm) | 0.65 | 0.90 | 0.80 | 0.80 | 0.70 |
| LENGTH OF EFFECT UPON TUMOR ON SACRIFICE DATE (cm) | 0.00 | 0.75 | 0.00 | 0.00 | 0.35 |
| WIDTH OF EFFECT UPON TUMOR ON SACRIFICE DATE (cm) | 0.00 | 0.80 | 0.00 | 0.00 | 0.90 |
| DEPTH OF EFFECT UPON TUMOR ON SACRIFICE DATE (cm) | 0.00 | 0.90 | 0.00 | 0.00 | 0.25 |
| COMMENTS AS RESULT OF TUMOR ASSESSMENT | no effect | not clear if the effect is due to the treatment | no effect | no effect | |

The results of Table XI - XII are summarized in Table XIII.

TABLE XIII

| Compound | Drug Dose mg/kg | Tumor Type | Time in Hrs. between Drug Introduction & Light Treatment | Position of Tumor in animal | Light Intensity in mW/cm² | Light Dose in J/cm² | Wavelength used to treat tumors in nm | Depth in cm of effect upon tumor | S.D. | n | P |
|---|---|---|---|---|---|---|---|---|---|---|---|
| L-α mono-serinyl chlorin $e_6$ | 100 | Smt-f | 24 | rt. leg | 75.0 | 20.0 | 665 | 0.53 ± | 0.09 | 9 | 0.45 |
| L-α monoglycl chlorin $e_6$ | 100 | Smt-f | 24 | rt. leg | 75.0 | 20.0 | 665 | 0.15 ± | 0.13 | 8 | 0.0 |

The preparation of pharmacological dosages for the administration of the active ingredient, that is the amino acid porphyrin adducts, which were prepared in Examples hereinabove, is as follows:

EXAMPLE 15

A tablet base was prepared by blending the following ingredient in the proportion by weight indicated:

| | Grams |
|---|---|
| Sucrose, USP | 80.3 |
| Tapioca Starch | 13.2 |
| Magnesium Stearate | 4.4 |

Into this base, there was blended sufficient amino acid porphyrin adducts to provide tablets each containing 100 mg. of active ingredient.

EXAMPLE 16

A blend was prepared containing the following ingredients:

| | |
|---|---|
| Calcium phosphate | 17.6 |
| Dicalcium phosphate | 18.8 |
| Magnesium trisilicate, USP | 5.2 |
| Lactose, U.S.P. | 5.2 |
| Potato Starch | 5.2 |
| Magnesium Stearate A | 0.8 |
| Magnesium Stearate B | 0.32 |
| Porphyrin Amino Acid Adducts | 20 |

This blend was divided and formed into capsules each containing 25 mg of active ingredient.

EXAMPLE 17

To a commercially available raspberry flavored sugar syrup is added the equivalent of 40 mg of the amino acid porphyrin adduct per milliliter and the mixture is homogenized in a mechanical device for this purpose. This mixture is especially suitable for oral administration containing 200 mg of the active ingredient.

EXAMPLE 18

A sterile solution of the following composition is prepared: 200 mg of the sodium salt of the amino acid porphyrin adduct is dissolved in a 0.9% NaCl solution so that the final concentration is 20 mg/ml.

This solution is suitable for I.V. and I.M. administration.

EXAMPLE 19

The sodium salt of the amino acid porphyrin adduct is dissolved in 0.9% NaCl solution so that the final concentration is 5 mg/ml. This is placed in an aerosal dispenser with a hydrocarbon propellant. This preparation is suitable for topical application.

EXAMPLE 20

PREPARATION OF A METAL SALT

The sodium salt of the porphyrin amino acid adduct is prepared by dissolving said adduct in water containing an equimolar amount of sodium hydroxide and freeze drying the resulting mixture.

In this fashion, other metal salts are prepared including potassium, calcium, and lithium salts.

PREPARATION OF AN ACID SALT

The amino acid porphyrin adduct described in the preceding examples are converted to acid salts, e.g., hydrochloride, by dissolving in an aqueous solution containing an equivalent amount of acid, e.g., hydrochloric acid, and the solution is evaporated to dryness to obtain the solid salt. Alternately, alcoholic solutions of hydrogen chloride gas, dissolved in ethanol can be used in lieu of the aqueous acid solution

and the acid salt in obtained by evaporation of the solvent or crystallization from the alcohol, e.g., by addition of a non-solvent.

**Claims**

**Claims for the following Contracting States: BE, CH, DE, FR, GB, IT, LI, LU, NL, SE**

**1.** A fluorescent mono-, di- or polyamide of an aminomonocarboxylic acid and a tetrapyrrole compound of the formula:

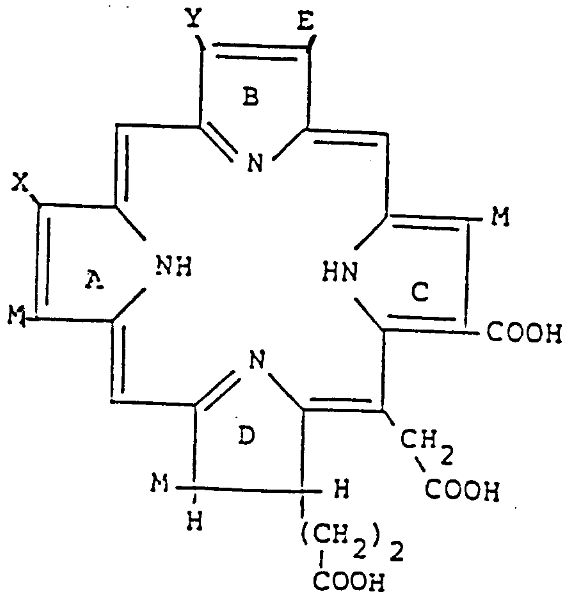

wherein

X = H, vinyl, ethyl or formyl;
Y = methyl or formyl;
M = methyl; and
E = ethyl

and pharmaceutically-acceptable salts thereof.

**2.** The amide according to Claim 1 wherein the amino acid is an alpha amino acid.

**3.** The amide according to Claims 1-2 wherein the amino acid is a polar alpha amino acid.

**4.** The amide according to Claim 1-3 wherein the carboxylic acid groups are asymmetrically attached to the tetrapyrrole ring system.

**5.** A mono- or diamide according to Claims 1-4.

**6.** The compound according to Claim 1 which is
mono- or di-L-serinyl chlorin $e_6$,
mono- or di-L-serinyl bacteriochlorin $e_6$,
mono- or di-L-cysteinyl bacteriochlorin $e_6$,
mono- or di-L-serinyl rhodin $g_7$,
mono- or di-L-cysteinyl rhodin $g_7$,
mono- or di-L-cysteinyl mesochlorin $e_6$,
mono- or di-L-serinyl mesochlorin $e_6$,
mono- or dialanyl chlorin $e_6$,
dileucyl chlorin $e_6$,
di-methionyl chlorin $e_6$,
mono- or diglycyl chlorin $e_6$,

mono- or dithreoninyl chlorin $e_6$
mono- or dicysteinyl chlorin $e_6$,
dityrosyl chlorin $e_6$,
diarginyl chlorin $e_6$,
dihistidyl chlorin $e_6$, or
mono- or di-ε-amino-caproyl chlorin $e_6$, or pharmaceutically acceptable salts thereof.

**7.** Use of a fluorescent mono, di- or polyamide of Claim 1 for preparing a pharmaceutical composition for photo-diagnosis of tumors with light of 360 to 760 nanometers and/or phototherapy of tumors with light of 600 to 800 nanometers.

**Claims for the following Contracting State: AT**

**1.** Use of a fluorescent mono-, di- or polyamide of an aminomonocarboxylic acid and a tetrapyrrole compound of the formula:

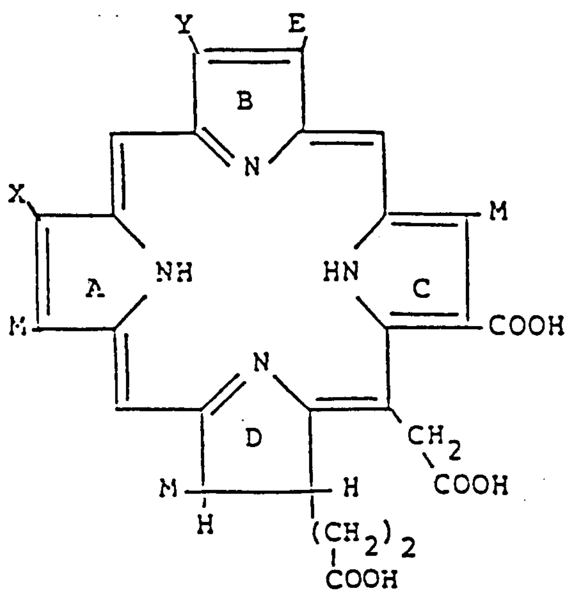

wherein
X = H, vinyl, ethyl or formyl;
Y = methyl or formyl;
M = methyl; and
E = ethyl
and pharmaceutically acceptable salts thereof for preparing a pharmaceutical composition for photodiagnosis of tumors with light of 360 to 760 nanometers and/or phototherapy of tumors with light of 600 to 800 nanometers.

**2.** Use according to claim 1, wherein the amino acid is an alpha amino acid.

**3.** Use according to claims 1 or 2, wherein the amino acid is a polar alpha amino acid.

**4.** Use according to claims 1 to 3, wherein the carboxylic acid groups are asymmetrically attached to the tetrapyrrole ring system.

**Revendications**
**Revendications pour les Etats contractants suivants: BE, CH, DE, FR, GB, IT, LI, LU, NL, SE**

**1.** Mono-, di- ou polyamide fluorescent d'un acide amino monocarboxylique et d'un composé de tétrapyrrole représenté par la formule :

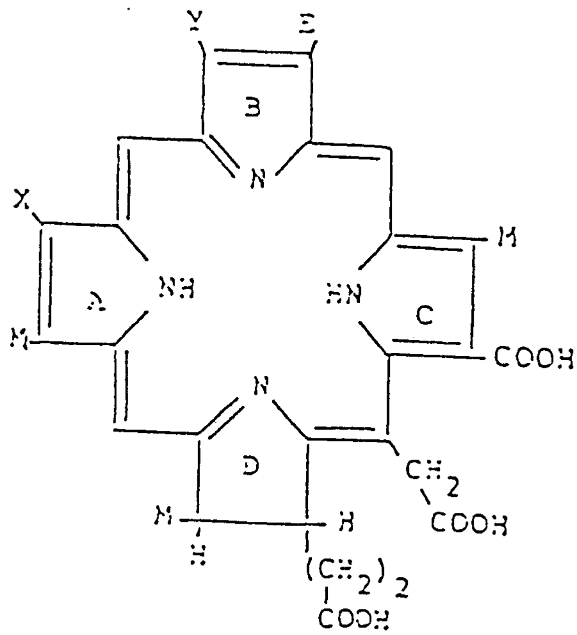

dans laquelle :

- X = H, vinyle, éthyle ou formyle ;
- Y = méthyle ou formyle ;
- M = méthyle ; et
- E = éthyle

et leurs sels pharmaceutiquement acceptables.

**2.** Amide selon la revendication 1, dans lequel l'acide aminé est un alpha amino acide.

**3.** Amide selon l'une des revendications 1 et 2, dans lequel l'acide aminé est un alpha amino acide polaire.

**4.** Amide selon l'une des revendications 1 à 3, dans lequel les groupes acide carboxylique sont fixés de manière asymétrique au système du noyau tétrapyrrole.

**5.** Mono- ou diamide selon l'une des revendications 1 à 4.

**6.** Composé selon la revendication 1, qui est :

- la mono- ou di-L-sérinyl chlorine $e_6$,
- la mono- ou di-L-sérinyl bactériochlorine $e_6$,
- la mono- ou di-L-cystéinyl bactériochlorine $e_6$,
- la mono- ou di-L-sérinyl rhodine $g_7$,
- la mono- ou di-L-cystéinyl rhodine $g_7$,
- la mono- ou di-L-cystéinyl mésochlorine $e_6$,
- la mono- ou di-L-sérinyl mésochlorine $e_6$,
- la mono- ou dialanyl chlorine $e_6$,
- la dileucyl chlorine $e_6$,
- la di-méthionyl chlorine $e_6$,
- la mono- ou diglycyl chlorine $e_6$,
- la mono- ou dithréoninyl chlorine $e_6$,
- la mono- ou dicystéinyl chlorine $e_6$,
- la dityrosyl chlorine $e_6$,
- la diarginyl chlorine $e_6$,
- la dihistidyl chlorine $e_6$, ou
- la mono- ou di-ε-amino-caproyl chlorine $e_6$,

ou leurs sels pharmaceutiquement acceptables.

7. Utilisation d'un mono-,di- ou polyamide fluorescent tel que défini à la revendication 1 pour la préparation d'une composition pharmaceutique pour le photodiagnostic de tumeurs avec une lumière de 360 à 760 nanomètres et/ou la photothérapie de tumeurs avec une lumière de 600 à 800 nanomètres.

**Revendications pour l'Etat suivant: AT**

1. Utilisation d'un mono-, di- ou polyamide fluorescent d'un acide amino monocarboxylique et d'un composé de type tétrapyrrole, représenté par la formule :

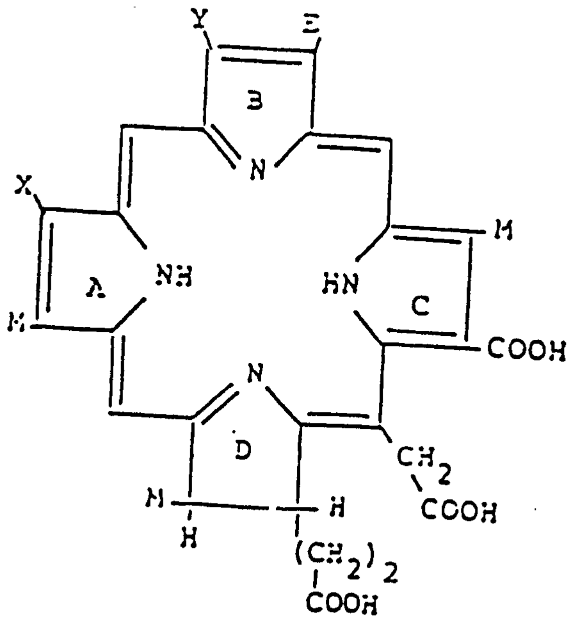

dans laquelle :
- X = H, vinyle, éthyle ou formyle ;
- Y = méthyle ou formyle ;
- M = méthyle ; et
- E = éthyle ; et

et de leurs sels pharmaceutiquement acceptables, pour la préparation d'une composition pharmaceutique pour le photodiagnostic de tumeurs avec une lumière de 360 à 760 nanomètres et/ou la photothérapie de tumeurs avec une lumière de 600 à 800 nanomètres.

2. Utilisation selon la revendication 1, dans laquelle l'acide aminé est un alpha amino acide.

3. Utilisation selon l'une des revendications 1 ou 2, dans laquelle l'acide aminé est un alpha amino acide polaire.

4. Utilisation selon l'une des revendications 1 à 3, dans laquelle les groupes acide carboxylique sont fixés de manière asymétrique au système de noyau tétrapyrrole.

**Patentansprüche**

**Patentansprüche für folgende Vertragsstaaten: BE, CH, DE, FR, GB, IT, LI, LU, NL, SE**

1. Fluoreszierendes Mono-, Di- oder Polyamid einer Aminomonocarbonsäure und einer Tetrapyrrolverbindung der Formel:

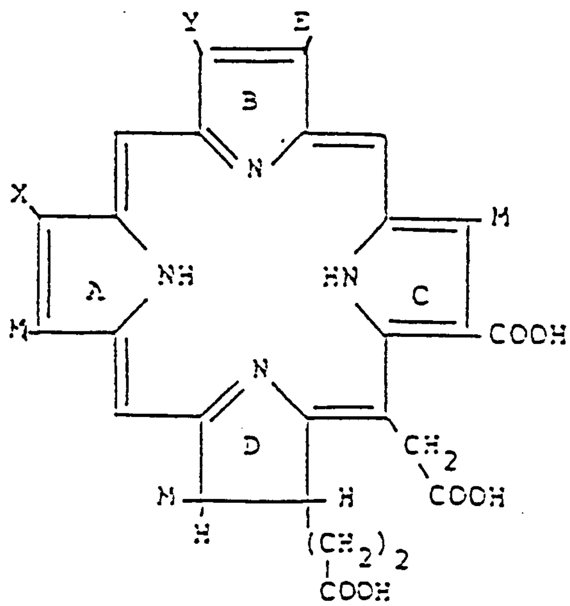

worin X = H, Vinyl, Ethyl oder Formyl; Y = Methyl oder Formyl; M = Methyl; und E = Ethyl, und pharmazeutisch verträgliche Salze davon.

**2.** Amid gemäß Anspruch 1, worin die Aminosäure eine alpha-Aminosäure ist.

**3.** Amid gemäß Anspruch 1 bis 2, worin die Aminosäure eine polare alpha-Aminosäure ist.

**4.** Amid gemäß Anspruch 1 bis 3, worin die Carboxylsäuregruppen asymmetrisch an das Tetrapyrrolringsystem gebunden sind.

**5.** Mono- oder Diamid gemäß Ansprüchen 1 bis 4.

**6.** Verbindung gemäß Anspruch 1, die
Mono- oder Di-L-serinylchlorin $e_6$
Mono- oder Di-L-serinylbakteriochlorin $e_6$
Mono- oder Di-L-cysteinylbakteriochlorin $e_6$
Mono- oder Di-L-serinylrhodin $g_7$
Mono- oder Di-L-cysteinaylrhodin $g_7$
Mono- oder Di-L-cysteinylmesochlorin $e_6$
Mono- oder Di-L-serinylmesochlorin $e_6$
Mono- oder Dialanylchlorin $e_6$
Dileucylchlorin $e_6$
Dimethionylchlorin $e_6$
Mono- oder Diglycylchlorin $e_6$
Mono- oder Dithreoninylchlorin $e_6$
Mono- oder Dicysteinylchlorin $e_6$
Dityrosylchlorin $e_6$
Diarginylchlorin $e_6$
Dihistidylchlorin $e_6$ oder
Mono- oder Di-epsilon-aminocaproylchlorin $e_6$ oder pharmazeutisch verträgliche Salze davon.

**7.** Verwendung eines fluoreszierenden Mono-, Di- oder Polyamids des Anspruchs 1 zur Herstellung eines pharmazeutischen Mittels zur Photodiagnose von Tumoren mit Licht von 360 bis 760 nm und/oder Phototherapie von Tumoren mit Licht von 600 bis 800 nm.

**Patentansprüche für folgenden Vertragsaat: AT**

1. Verwendung eines fluoreszierenden Mono-, Di- oder Polyamids einer Aminomonocarbonsäure und einer Tetrapyrrolverbindung der Formel:

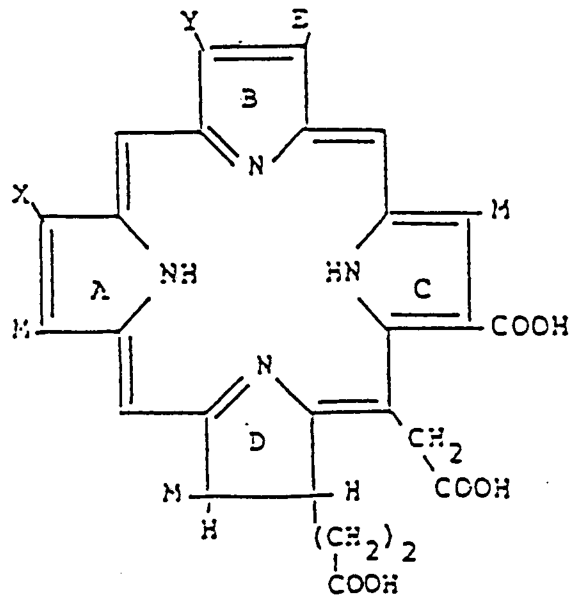

worin X = H, Vinyl, Ethyl oder Formyl; Y = Methyl oder Formyl; M = Methyl; und E = Ethyl, und eines pharmazeutisch verträglichen Salzes davon zur Herstellung eines pharmazeutischen Mittels zur Photodiagnose von Tumoren mit Licht von 360 bis 760 nm und/oder zur Phototherapie von Tumoren mit Licht von 600 bis 800 nm.

2. Verwendung gemäß Anspruch 1, worin die Aminosäure eine alpha-Aminosäure ist.

3. Verwendung gemäß Anspruch 1 bis 2, worin die Aminosäure eine polare alpha-Aminosäure ist.

4. Verwendung gemäß Anspruch 1 bis 3, worin die Carboxylsäuregruppen asymmetrisch an das Tetrapyrrolringsystem gebunden sind.